(19) Europäisches Patentamt
European Patent Office
Office européen des brevets

(11) Publication number: 0 055 043 B1

(12) **EUROPEAN PATENT SPECIFICATION**

(45) Date of publication of patent specification: **23.01.85**

(21) Application number: **81305723.9**

(22) Date of filing: **04.12.81**

(51) Int. Cl.[4]: **C 07 D 209/60**, C 07 C 87/64, C 07 C 93/12, C 07 C 131/00, A 61 K 31/40

# (54) 6,7,8,9-Tetrahydro-3H-benz(e)indolamine derivatives, processes for their preparation and intermediates used therein, and pharmaceutical compositions containing the indolamine derivatives.

(30) Priority: **11.12.80 US 215482**
**19.10.81 US 312464**

(43) Date of publication of application:
**30.06.82 Bulletin 82/26**

(45) Publication of the grant of the patent:
**23.01.85 Bulletin 85/04**

(84) Designated Contracting States:
**AT BE CH DE FR GB IT LI LU NL SE**

(56) References cited:
**DE-A-2 044 172**
**DE-A-2 740 836**
**US-A-4 110 339**
**US-A-4 212 804**

**CHEMICAL ABSTRACTS, vol. 69, no. 25, December 16, 1968, Columbus, Ohio, USA, M. MOTTET "Condensations products of methyl dioxosuccinate with certain N-aryl beta-naphthylamines" page 9960, column 1, abstract no. 106400v**

(73) Proprietor: **AYERST, MCKENNA AND HARRISON INC.**
**1025 Laurentian Boulevard**
**St. Laurent Quebec H4R 1J6 (CA)**

(72) Inventor: **Asselin, Andre Alfred**
**1805, Muir Street**
**St. Laurent Quebec H4L 4T2 (CA)**
Inventor: **Humber, Leslie George**
**75 Maple Circle**
**Dollard des Ormeaux Quebec H9B 1E6 (CA)**

(74) Representative: **Wileman, David Francis et al**
**c/o John Wyeth and Brother Limited**
**Huntercombe Lane South**
**Taplow Maidenhead Berkshire SL6 OPH (GB)**

**EP 0 055 043 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to novel 6,7,8,9-tetrahydro-3H-benz[e]indolamine derivatives, to therapeutically acceptable acid addition salts thereof, to processes for their preparation and intermediates used therein, to methods of using the derivatives and to pharmaceutical compositions of the derivatives. These derivatives exhibit dopamine-receptor stimulating activity in a mammal. Thus, they can be useful for treating hyperprolactinemia, galactorrhea, amenorrhea, impotence, Parkinsonism, diabetes, acromegaly, hypertension and other central nervous system disorders which respond to dopamine-receptor stimulation.

A number of 6,7,8,9-tetrahydro-3H-benz[e]indole derivatives are known and described, for example, L. B. Shagalov *et al.*, Chem. Abstr., *91*, 56747 v (1979) for Khim. Geterotsikl. Soedin., (3), 360 (1979); L. B. Shagalov *et al.*, Chem. Abstr., *89*, 146703 r (1978) for Khim. Geterotsikl. Soedin., (5), 634 (1978); Derwent Publications Ltd., Farmdoc 46000U for Netherland Patent 7,300,871, published July 30, 1973; and Derwent Publications Ltd., Farmdoc 24087B for German Offenlegungsschrift 2,740,836, published March 22, 1979. The reported compounds lack the substituents on the 6,7,8,9-tetrahydro-3H-benz[e]indole ring system which are characteristic of the compounds of this invention. N. J. Bach and E. C. Kornfeld, U.S. Patent 4,110,339, August 29, 1978 disclose tricyclic tetrahydro-2H-benzo[c]pyrroles which are dopamine agonist. These latter compounds are distinguished most readily from the compounds of this invention by having a perifused tricyclic ring system.

The compounds of this invention are represented by formula I

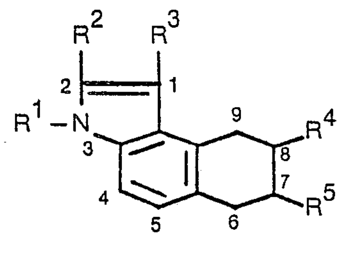

(I)

in which $R^1$, $R^2$ and $R^3$ each is hydrogen or lower alkyl having 1 to 5 carbon atoms, $R^4$ is $NR^6R^7$ wherein $R^6$ and $R^7$ each is hydrogen or lower alkyl having 1 to 5 carbon atoms and $R^5$ is hydrogen, or $R^4$ is hydrogen and $R^5$ is $NR^6R^7$ wherein $R^6$ and $R^7$ are as defined herein, or a therapeutically acceptable acid addition salt thereof.

A preferred group of compounds of this invention is represented by formula I in which $R^1$, $R^2$ and $R^3$ each is hydrogen or lower alkyl having 1 to 3 carbon atoms, $R^4$ is $NR^6R^7$ wherein $R^6$ and $R^7$ each is hydrogen or lower alkyl having 1 to 3 carbon atoms and $R^5$ is hydrogen, or $R^4$ is hydrogen and $R^5$ is $NR^6R^7$ wherein $R^6$ and $R^7$ each is hydrogen or lower alkyl having 1 to 3 carbon atoms, or a therapeutically acceptable acid addition salt thereof.

Another preferred group of compounds of this invention is represented by formula I in which $R^1$, $R^2$ and $R^3$ are hydrogen, $R^4$ is $NR^6R^7$ wherein $R^6$ and $R^7$ each is hydrogen or lower alkyl having 1 to 5 carbon atoms and $R^5$ is hydrogen, or $R^4$ is hydrogen and $R^5$ is $NR^6R^7$ wherein $R^6$ and $R^7$ each is hydrogen or lower alkyl having 1 to 5 carbon atoms, or a therapeutically acceptable acid addition salt thereof.

A most preferred group of compounds of this invention is represented by formula I in which $R^1$, $R^2$ and $R^3$ are hydrogen, $R^4$ is $NR^6R^7$ wherein $R^6$ and $R^7$ each is lower alkyl having 1 to 3 carbon atoms and $R^5$ is hydrogen, or $R^4$ is hydrogen and $R^5$ is $NR^6R^7$ wherein $R^6$ and $R^7$ each is lower alkyl having 1 to 3 carbon atoms, or a therapeutically acceptable acid addition salt thereof.

A pharmaceutical composition is provided by admixing the compound of formula I, or a therapeutically acceptable acid addition salt thereof, with a pharmaceutically acceptable carrier.

The compounds of this invention are used to stimulate dopamine receptors in a mammal in need thereof by administering to the mammal an effective dopamine receptor stimulating amount of a compound of formula I or a therapeutically acceptable acid addition salt thereof. The compounds of this invention are favorably used in combination with an effective amount of an agent commonly used in the treatment of Parkinsonism and related disorders, particularly those selected from bromocriptine, lergotrile, levodopa, combination of levodopa and carbidopa, L-prolyl-L-leucylglycinamide and L-propyl-N-methyl-D-leucylglycinamide.

The compounds of formula I or a therapeutically acceptable acid addition salt thereof can be prepared by one of the following:

(a) when a compound of formula I in which $R^1$, $R^2$ and $R^3$ are hydrogen, $R^4$ is $NR^6R^7$ wherein $R^6$ and $R^7$ each is lower alkyl having 1 to 5 carbon atoms and $R^5$ is hydrogen, or $R^4$ is hydrogen and $R^5$ is $NR^6R^7$ wherein $R^6$ and $R^7$ each is lower alkyl having 1 to 5 carbon atoms is required, reducing a corresponding compound of formula X

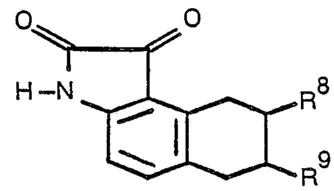

(X)

in which $R^8$ is $NR^{10}R^{11}$ wherein $R^{10}$ and $R^{11}$ each is lower alkyl and $R^9$ is hydrogen, or $R^8$ is hydrogen and $R^9$ is $NR^{10}R^{11}$ wherein $R^{10}$ and $R^{11}$ each is lower alkyl with a complex metal hydride;

(b) when a compound of formula I in which $R^1$ is hydrogen or lower alkyl, $R^2$ and $R^3$ are hydrogen, $R^4$ is $NR^6R^7$ wherein $R^6$ is hydrogen and $R^7$ is hydrogen or lower alkyl and $R^5$ is hydrogen, or $R^4$ is hydrogen and $R^5$ is $NR^6R^7$ wherein $R^6$ is hydrogen and $R^7$ is hydrogen or lower alkyl is required, hydrogenating a compound of formula XI

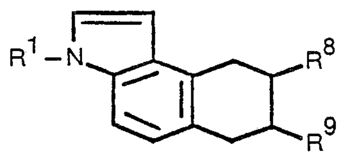

(XI)

in which $R^1$ is hydrogen or lower alkyl, $R^8$ is $NR^{10}R^{11}$ wherein $R^{10}$ is benzyl and $R^{11}$ is benzyl or lower alkyl and $R^9$ is hydrogen, or $R^8$ is hydrogen and $R^9$ is $NR^{10}R^{11}$ wherein $R^{10}$ is benzyl and $R^{11}$ is benzyl or lower alkyl;

(c) when a compound of formula I in which $R^1$ is hydrogen, $R^2$ is lower alkyl, $R^3$ is hydrogen or lower alkyl, $R^4$ is $NR^6R^7$ wherein $R^6$ and $R^7$ each is lower alkyl and $R^5$ is hydrogen, or $R^4$ is hydrogen and $R^5$ is $NR^6R^7$ wherein $R^6$ and $R^7$ each is lower alkyl is required, condensing a compound of formula XII

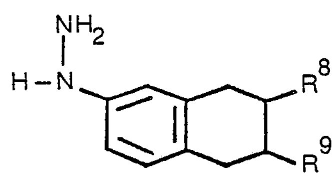

(XII)

in which $R^8$ is $NR^{10}R^{11}$ wherein $R^{10}$ and $R^{11}$ each is lower alkyl and $R^9$ is hydrogen, or $R^8$ is hydrogen and $R^9$ is $NR^{10}R^{11}$ wherein $R^{10}$ and $R^{11}$ each is lower alkyl with a ketone of the formula

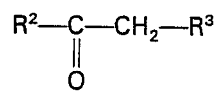

in which $R^2$ is lower alkyl and $R^3$ is hydrogen or lower alkyl according to the Fischer indole method;

(d) when a compound of formula I in which $R^1$ and $R^3$ each is hydrogen or lower alkyl, $R^2$ is lower alkyl, $R^4$ is $NR^6R^7$ wherein $R^6$ is hydrogen and $R^7$ is hydrogen or lower alkyl and $R^5$ is hydrogen, or $R^4$ is hydrogen and $R^5$ is $NR^6R^7$ wherein $R^6$ is hydrogen and $R^7$ is hydrogen or lower alkyl is required, hydrogenating a corresponding compound of formula XIV

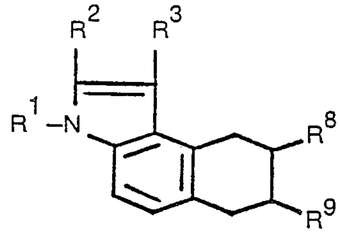

(XIV)

in which $R^1$ and $R^3$ each is hydrogen or lower alkyl, $R^2$ is lower alkyl, $R^8$ is $NR^{10}R^{11}$ wherein $R^{10}$ is benzyl

and $R^{11}$ is benzyl or lower alkyl and $R^9$ is hydrogen, or $R^8$ is hydrogen and $R^9$ is $NR^{10}R^{11}$ wherein $R^{10}$ is benzyl and $R^{11}$ is benzyl or lower alkyl;

(e) when a compound of formula I in which $R^1$ and $R^2$ are hydrogen, $R^3$ is lower alkyl, $R^4$ is $NR^6R^7$ wherein $R^6$ and $R^7$ each is lower alkyl and $R^5$ is hydrogen, or $R^4$ is hydrogen and $R^5$ is $NR^6R^7$ wherein $R^6$ and $R^7$ each is lower alkyl is required, decarboxylating a corresponding compound of formula XVI

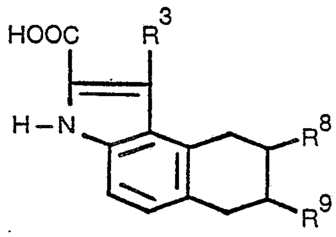

(XVI)

in which $R^3$ is lower alkyl, $R^8$ is $NR^{10}R^{11}$ wherein $R^{10}$ and $R^{11}$ each is lower alkyl and $R^9$ is hydrogen, or $R^8$ is hydrogen and $R^9$ is $NR^{10}R^{11}$ wherein $R^{10}$ and $R^{11}$ each is lower alkyl;

(f) when a compound of formula I in which $R^1$ is hydrogen or lower alkyl, $R^2$ is hydrogen, $R^3$ is lower alkyl, $R^4$ is $NR^6R^7$ wherein $R^6$ is hydrogen and $R^7$ is hydrogen or lower alkyl and $R^5$ is hydrogen, or $R^4$ is hydrogen and $R^5$ is $NR^6R^7$ wherein $R^6$ is hydrogen and $R^7$ is hydrogen or lower alkyl is required, hydrogenating a corresponding compound of formula XVII

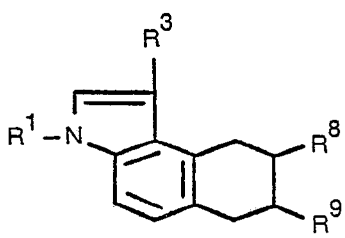

(XVII)

in which $R^1$ is hydrogen or lower alkyl, $R^3$ is lower alkyl, $R^8$ is $NR^{10}R^{11}$ wherein $R^{10}$ is benzyl and $R^{11}$ is benzyl or lower alkyl and $R^9$ is hydrogen, or $R^8$ is hydrogen and $R^9$ is $NR^{10}R^{11}$ wherein $R^{10}$ is benzyl and $R^{11}$ is benzyl or lower alkyl;

(g) when a compound of formula I in which $R^1$ is lower alkyl, $R^2$ and $R^3$ each is hydrogen or lower alkyl, $R^4$ is $NR^6R^7$ wherein $R^6$ and $R^7$ each is lower alkyl and $R^5$ is hydrogen, or $R^4$ is hydrogen and $R^5$ is $NR^6R^7$ wherein $R^6$ and $R^7$ each is lower alkyl, is required, alkylating the corresponding compound of formula I in which $R^1$ is hydrogen, $R^2$ and $R^3$ each is hydrogen or lower alkyl, $R^4$ is $NR^6R^7$ wherein $R^6$ and $R^7$ each is lower alkyl and $R^5$ is hydrogen, or $R^4$ is hydrogen and $R^5$ is $NR^6R^7$ wherein $R^6$ and $R^7$ each is lower alkyl, and

(h) when a therapeutically acceptable acid addition salt of a compound of formula I is required, reacting the compound of formula I with a therapeutically acceptable acid.

The intermediates of formula X as defined above and processes for preparing them are also within the scope of this invention.

The term "lower alkyl" as used herein means straight and branched chain alkyl radicals containing from one to five carbon atoms and includes methyl, ethyl, propyl, 1-methylethyl, butyl, 1,1-dimethylethyl and pentyl, unless stated otherwise.

The term "complex metal hydride" as used herein means metal hydride reducing agents and includes, for example, lithium aluminum hydride, lithium aluminum hydride-aluminum chloride, aluminum hydride-aluminum chloride, diborane, diisobutylaluminum hydride, borane methyl sulfide and sodium borohydride-aluminum chloride.

Also included in this invention are the stereochemical isomers of the compounds of formula I which result from asymmetric centers contained therein. These isomeric forms may be prepared by chemical methods and are purified readily by crystallization or chromatography.

Individual optical isomers, which might be separated by fractional crystallization of the diastereoisomeric salts formed thereof, for instance, with d- or l-tartaric acid or D-(+)-$\alpha$-bromocamphor sulfonic acid, are also included.

The compounds of formula I are capable of forming acid addition salts with therapeutically acceptable acids. The acid addition salts are prepared by reacting the base form of the appropriate compound of formula I with one or more equivalents, preferably with an excess, of the appropriate acid in an organic solvent, for example, diethyl ether or an ethanol-diethyl ether mixture. These salts, when

administered to a mammal, possess the same pharmacologic activities as the corresponding bases. For many purposes it is preferable to administer the salts rather than the base compounds. Examples of suitable acids to form these salts include: the common mineral acids, e.g. hydrohalic, sulfuric or phosphoric acids; the organic acids, e.g., formic, acetic, maleic, methanesulfonic, malic, citric, or tartaric acid; and acids which are sparingly soluble in body fluids and which impart slow-release properties to their respective salts, e.g., pamoic acid, tannic acid or carboxymethyl cellulose. The addition salts thus obtained are the functional equivalent of the parent base compound in respect to their therapeutic use. Hence, these addition salts are included within the scope of this invention and are limited only by the requirement that the acids employed in forming the salts be therapeutically acceptable.

The discovery in the mid-1960's of two major dopamine (DA) systems indicated that this neurotransmitter exerted control over a number of physiological functions. Against this background an interest arose to develop DA receptor agonists to study the function of the dopaminergic systems and to evaluate these agonists as possible therapeutic agents in Parkinson's disease and certain neuroendocrine disorders, for example, hyperprolactinemia, galactorrhea, amenorrhea, impotence, hypertension and other central nervous system disorders.

The DA receptor agonists exert a variety of pharmacological effects, some of the most characteristic being the ones that occur in animals in which DA deficiency is brought about to mimic the Parkinsonian syndrome. An important model was developed by U. Ungerstedt, Acta. Physiol. Scand., Suppl. 367, 69—93 (1971) who, by means of unilateral injections of 6-hydroxy-dopamine (6-OHDA) into the DA pathway, could product selective lesions of the ascending DA pathways on one side of the brain. Ungerstedt (1971) demonstrated in these lesioned rats that DA receptor agonists induced rotational behavior towards the innervated side. The response is due to the development of receptor supersensitivity in the denervated striatum resulting in a higher degree of DA receptor activity on the denervated- as compared to the innervated-side after treatment with DA receptor agonists. Due to this imbalance between the two sides, a rotational behavior is elicited, the direction being always towards the less activated side. It is of interest that in the discovery of the DA receptor stimulating properties of bromocriptine, the 6-OHDA rotational model was utilized [H. Corrodi *et al.*, J. Pharm. Pharmacol., *25*, 409—412 (1973)].

In the test for rotational behavior in rats following the unilateral 6-OHDA-induced destruction of one nigrostriatal pathway, the method described by C. J. Pycock and C. D. Marsden, Europ. J. Pharmacol., *47*, 167 (1978) was followed. The rats (230—250 g) were anesthetized with sodium pentobarbital (40 mg/kg i.p.) and intracerebral injections were made using a Stoelting stereo-taxic instrument, (C. H. Stoelting Co., Chicago, Ill., U.S.A.). Unilateral injections of 6-OHDA hydrobromide (8 $\mu$g/3 $\mu$l delivered at a rate of 1 $\mu$l per min) were made into the ascending median forebrain bundle (MFB) in the lateral hypo-made into the ascending median forebrain bundle (MFB) in the lateral hypothalamus according to the coordinates of the De Groot brain atlas, J. De Groot, Verhandel, Koninkl. Ned. Akad. Wetenschap. Natuurk. *52*: 1—40 (1959), (A: +4.6, L: $\pm$ 1.9, V: −2.7). 6-OHDA was made up in ice-cold distilled water containing 0.2 mg/ml ascorbic acid.

Three weeks after operation, the rats were tested for rotational behavior in response to apomorphine hydrochloride (0.25 mg/kg, s.c.). Rats which consistently showed more than 5 turns/min. after apomorphine were selected and the compound of formula I was then administered. The rat was immediately placed in the rotometer, described by K. Voith and J. R. Cummings, Can. J. Pharmacol., *54*, 551 (1976), and the rotation was continuously recorded until drug effect subsided. In this test, the following compounds of formula I are demonstrated to be an effective dopamine receptor agonist [the amount of the compound, route of administration and total turns $\pm$ S.E. (standard error) during the time observed are indicated in the parenthesis]: 6,7,8,9-tetrahydro-N,N-dipropyl-3H-benz[e]indol-7-amine (as a subcutaneous dose of 2.5 mg/kg exhibited 2867 $\pm$ 269 turns in an 7.5 hour duration) and 6,7,8,9-tetrahydro-N,N-diproyl-3H-benz[e]indol-8-amine (at a subcutaneous dose of 5 mg/kg exhibited 3554 $\pm$ 310 turns in an eight hour duration).

A recently developed animal model, described by G. P. Smith and R. C. Yound in "Advances in Neurology", Vol. 5, F. H. McDowell and A. Barbeau, Eds., Raven Press, New York, pp. 427—432 (1974), shows that rats exhibit almost complete akinesia in an open field following the bilateral injection of 6-OHDA into the anterolateral hypothalamus. The compounds of formula I can reverse this 6-OHDA-induced hypokinesia as a result of their functioning as dopamine receptor agonists. In this test for dopamine receptor agonists, the compounds of formula I can exhibit a pharmacologial response that is comparable to that of apomophine and bromocriptine.

Experiments are performed on male Sprague-Dawley rats housed in air-conditioned quarters. The room is lighted between 0700 and 1900 hr daily and maintained at a temperature of 24°C $\pm$ 2°C.

The method of Smith and Young, cited above, is followed. Rats (approximately 280 g) are operated on under sodium pentobarbital anesthesia. Using a Stoelting stereotaxic instrument, the top of a 26 gauge cannula is positioned in the anterolateral hypothalamus (7 mm anterior to the interaural line, 2 mm lateral to the midline and 8 mm below the dura) according to the De Groot brain atlas, noted above. Via a polyethylene tubing (PE 20), the cannula is connected to a 10 $\mu$l syringe which is mounted in a Starrett micrometer head drive, C. H. Stoelting Co., Chicago, Illinois, U.S.A. All injections are

bilateral. Each injection consisted of 4 $\mu$l of distilled water containing 6-OHDA (6.5 $\mu$g base/$\mu$l) and ascorbic acid (0.4 $\mu$g/$\mu$l).

The animals have free access to Purina Laboratory Chow pellets and tap water. However since anterolateral hypothalamic 6-OHDA injections produce aphagia and adipsia, intragastric feeding is necessary in order to prevent drastic weight loss. The rats receive a daily gastric intubation of 2 g of the "modified rat tube feeding diet" (ICN Pharmaceuticals, Inc., Cleveland, Ohio, U.S.A.) mixed with approximately 2 ml tap water.

Ambulation in the open field is evaluated in an apparatus consisting of a wooden box (69 cm × 69 cm × 42 cm) with an arborite floor. The floor is divided into 36 squares (11.5 cm × 11.5 cm). The placement of all four limbs in one square is taken as one ambulation score.

In the present experiments all compounds are evaluated four days after the intracerebral injection of 6-OHDA. The rat is placed into the center of the open field and observed for a 2-min. period. Only rats with almost total akinesia are used. Apomorphine, bromocriptine or the compounds of formula I are injected s.c. to groups of 4—12 rats. Subsequently, the number of squares are counted which the animal entered during several 2-min observation periods. Apomorphine is evaluated at 5, 10, 15, 20 and 30 min; bromocriptine at 2, 3, 4, 5, 6 and 7 hr; and the compounds of formula I at 15, 30, 45, 60, 90 and 120 min after injection. Each animal is used only once. The results are expressed as cumulative number of ambulation scores, which are the sums of the scores obtained during the 2-min observation periods.

The following substances are used; apomorphine hydrochloride (Macfarlan Smith Ltd., Edinburgh, Scotland), bromocriptine (CB-154) (Sandoz Pharmaceuticals, East Hanover, N.J., U.S.A.) and 6-OHDA hydrobromide (Aldrich Chemical Co., Inc., Milwaukee, Wisconsin, U.S.A.). The compounds are dissolved in distilled water or suspended in distilled water with a few drops of polysorbate 80 (Tween 80; "Tween" is a registered trade mark). If the compound is an oil, 0.4 ml of dimethyl sulfoxide is added. Solutions are prepared fresh on the day of the experiment. The 6-OHDA solution is kept in ice throughout the injection procedure. All doses refer to the base.

Using the above described method, apomorphine at a dose of 0.5 mg/kg exhibits a score of 135 $\pm$ 41 and bromocriptine at a dose of 10 mg/kg exhibits a score of 112 $\pm$ 23. Similarly, the following compound of formula 1 is an effective dopamine receptor agonist, 6,7,8,9-tetrahydro-N,N-dipropyl-3H-benz[e]indol-7-amine (at a subcutaneous dose of 2.5 mg/kg of body weight exhibited a cumulative ambulation score of 155 $\pm$ 28), 6,7,8,9-tetrahydro-N,N-dipropyl-3H-benz[e]indol-8-amine (at a subcutaneous dose of 2.5 mg/kg of body weight exhibited a cumulative ambulation score of 287 $\pm$ 109), and 6,7,8,9-tetrahydro-3-methyl-N,N-dipropyl-3H-benz[e]indol-8-amine (at a subcutaneous dose of 10 mg/kg of body weight exhibited a cumulative ambulation score of 68 $\pm$ 34).

The above described test methods for dopamine receptor agonists show that the compounds of formula I are active as dopamine receptor agonists. The compounds, thus, can be used clinically in the treatment of hyperprolactinemia, galactorrhoea, amenorrhoea, impotence, diabetes, Parkinsonism, acromegaly, hypertension and other central nervous system disorders, which respond to dopamine-receptor stimulation.

The compounds of formula I of this invention are used alone or in combination with pharmacologically acceptable carriers, the proportion of which is determined by the solubility and chemical nature of the compound, chosen route of administration and standard biological practice. For example, they are administered orally in solid form i.e. capsule or tablet. They can also be administered orally in the form of suspensions or solutions or they may be injected parenterally. For parenteral administration they can be used in the form of a sterile solution containing other solutes, for example, enough saline or glucose to make the solution isotonic.

The tablet compositions contain the active ingredient in admixture with non-toxic pharmaceutical excipients known to be suitable in the manufacture of tablets. Suitable pharmaceutical excipients are, for example, starch, milk sugar, certain types of clay and so forth. The tablets can be uncoated or they can be coated by known techniques so as to delay disintegration and absorption in the gastrointestinal tract and thereby provide a sustained action over a longer period.

The aqueous suspensions of the compounds of formula I contain the active ingredient in admixture with one or more non-toxic pharmaceutical excipients known to be suitable in the manufacture of aqueous suspensions. Suitable excipients are, for example, methylcellulose, sodium alginate, gum acacia, lecithin and so forth. The aqueous suspensions can also contain one or more preservatives, one or more coloring agents, one or more flavoring agents and one or more sweetening agents.

Non-aqueous suspensions can be formulated by suspending the active ingredient in a vegetable oil, for example, arachis oil, olive oil, sesame oil, or coconut oil, or in a mineral oil, for example liquid paraffin, and the suspension may contain a thickening agent, for example beeswax, hard paraffin or cetyl alcohol. These compositions can also contain a sweetening agent, flavoring agent and antioxidant.

The dosage of the compounds of formula I as dopamine receptor agonists will vary with the form of administration and the particular compound chosen. Furthermore, it will vary with the particular host as well as the age, weight and condition of the host under treatment as well as with the nature and extent of the symptoms. Generally, treatment is initiated with small doses substantially less than the

optimum dose of the compound. Thereafter, the dosage is increased by small increments until the optimum effect under the circumstances is reached. In general, the compounds of this invention are most desirably administered at a concentration level that will generally afford effective results without causing any harmful or deleterious side effects. For example, the effective dopamine receptor stimulating amount of the compounds for i.p. administration usually ranges from about 0.1 mg to about 250 mg per kilogram body weight per day in single or divided doses although as aforementioned variations will occur. However a dosage level that is in the range of from about 0.1 to about 100 mg per kilogram body weight per day in single or divided doses is employed most desirably for i.p. administration in order to achieve effective results. For oral administration, effective amounts can range from about 0.5 to about 250 mg per kilogram body weight per day in single or divided doses preferably about 1.0 to 50 mg per kilogram of body weight per day.

The compound of formula I, or a therapeutically acceptable salt thereof, also can be used to produce beneficial effects in the treatment of Parkinsonism, hyperprolactinemia and related disorders when combined with a therapeutically effective amount of an agent commonly used in the treatment of Parkinsonism, hyperprolactinemia and related disorders. Such agents include, for example, apomorphine and its derivatives, piribedil and its derivatives, dopaminergic ergot derivatives, especially bromocriptine and lergotrile, 2-amino-6,7-dihydroxy-(1,2,3,4)-tetrahydronaphthalene (ADTN), levo-dihydroxyphenylalanine (levodopa), combination of levodopa with carbidopa, L-prolyl-L-leucylglycinamide (MIF) and its derivatives, especially L-prolyl-N-methyl-D-leucylglycinamide (pareptide), biperiden, cycrimine hydrochloride, procyclidine, trihexyphenidyl hydrochloride, benztropine mesylate, chlorphenoxamine hydrochloride, diphenhydramine hydrochloride, orphenadrine hydrochloride, ethopropazine hydrochloride and the enzymes, monoamine oxidase B and catechol-O-methyl transferase. A combination of the foregoing agents can be substituted for a single agent. Suitable methods of administration, compositions and dosages of the agents are well known in the art; for instance, "Physican Desk Reference", 32 ed., Medical Economics Co., Oradell, N.J., U.S.A., 1978. When used in combination, the compound of formula I, or its therapeutically acceptable salt, is administered as described previously.

*Process*

Reaction scheme 1 illustrates a method of preparing a diamine of formula II, an intermediate for the preparation of the compounds of formula I.

REACTION SCHEME 1

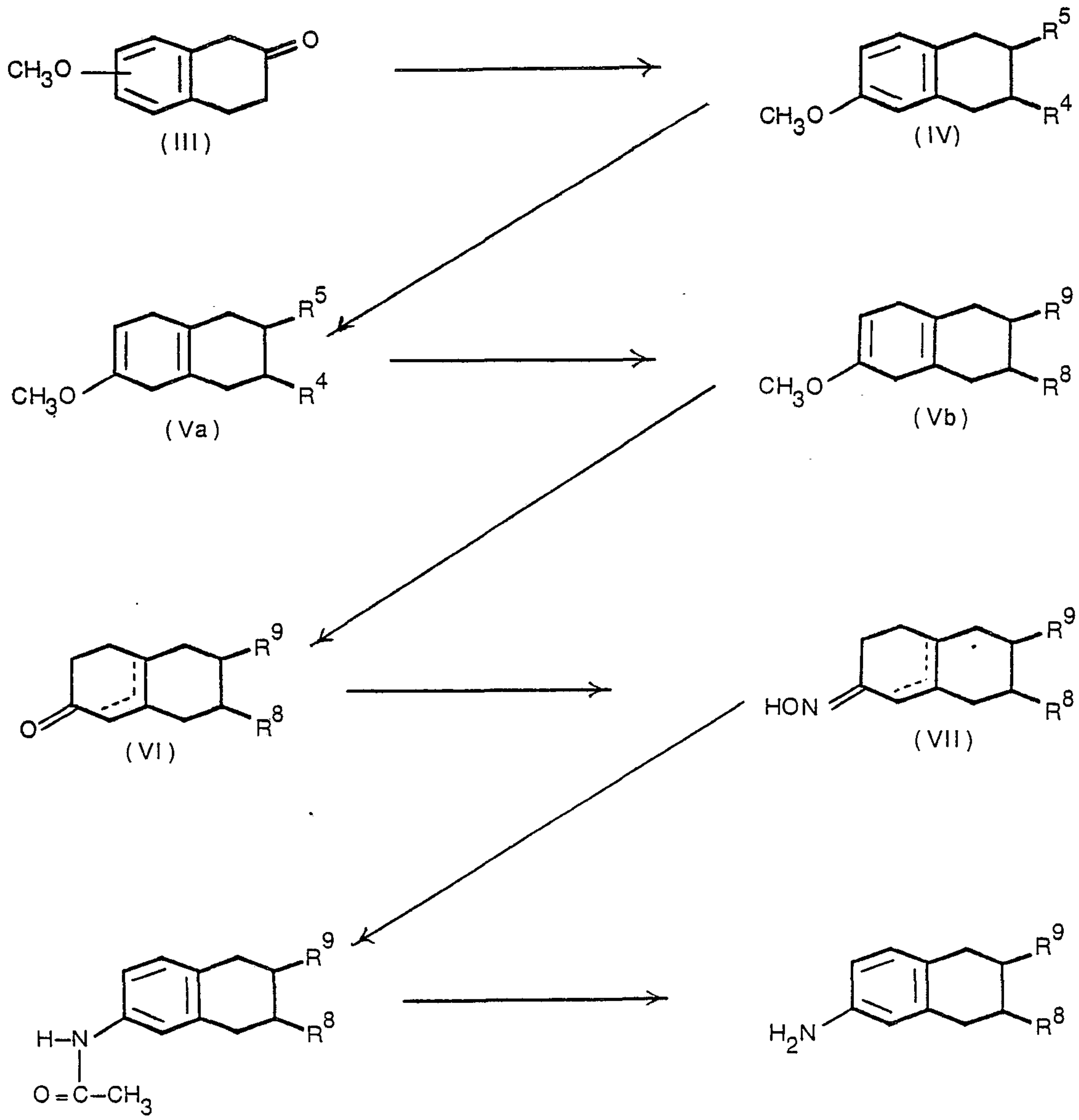

(III) (IV) (Va) (Vb) (VI) (VII) (VIII) (II)

The starting material for the preparation of the intermediate of formula II is the 6- or 7-methoxy-2-tetralone of formula III. The compound of formula III is reacted with an excess of a di(lower alkyl) amine and about a molar amount of p-toluenesulfonic acid in an inert organic solvent, for example, benzene, at about 70 to 90°C for about one to five days and concurrent removal of water by azeotropic distillation. Reduction of the resulting enamine with hydrogen (about one to three atmospheres of pressure) in the presence of platinum oxide in ethanol gives the corresponding compound of formula IV in which $R^4$ or $R^5$ is $NR^6R^7$ wherein $R^6$ and $R^7$ each is lower alkyl. Similarly, reaction of the compound of formula III with a lower alkyl amine, and reduction with sodium borohydride or catalytic hydrogenation of the resulting enamine gives the corresponding compound of formula IV in which $R^4$ or $R^5$ is $NR^6R^7$ wherein $R^6$ is hydrogen and $R^7$ is lower alkyl. Another compound of formula IV is obtained by reaction of the compound of formula III with hydroxylamine to obtain the oxime, followed by reduction of the oxime with lithium aluminum hydride or nickel aluminum alloy to give the corresponding compound of formula IV in which $R^4$ or $R^5$ is $NR^6R^7$ wherein $R^6$ and $R^7$ are hydrogen. The latter compound of formula IV in whcih $R^6$ and $R^7$ are hydrogen can also be prepared by reacting the compound of formula III with benzylamine to obtain the corresponding enamine, followed by reduction of the enamine with hydrogen, in the same manner as described above.

Alternatively, the compound of formula IV in which $R^4$ or $R^5$ is $NR^6R^7$ wherein $R^6$ is hydrogen and $R^7$ is hydrogen or lower alkyl can be reacted with a N-alkylating agent to give the compound of formula IV in which $R^4$ or $R^5$ is $NR^6R^7$ wherein $R^6$ and $R^7$ each is lower alkyl.

Birch reduction of the compound of formula IV in which $R^4$ and $R^5$ are as defined herein with sodium, e.g. in a solution of tetrahydrofuran, isopropanol and liquid ammonia at about −70°C gives the corresponding compound of formula Va in which $R^4$ or $R^5$ are as defined herein. Reaction of the compound of formula Va in which $R^4$ or $R^5$ is $NR^6R^7$ wherein $R^6$ is hydrogen and $R^7$ is hydrogen or lower alkyl with benzyl bromide, chloride or iodide gives the corresponding compound of formula Vb in which $R^8$ or $R^9$ is $NR^{10}R^{11}$ wherein $R^{10}$ is benzyl and $R^{11}$ is benzyl or lower alkyl. The compound of formula Vb in which $R^8$ or $R^9$ is $NR^{10}R^{11}$ wherein $R^{10}$ and $R^{11}$ each is lower alkyl is equivalent to the compound of formula Va in which $R^4$ or $R^5$ is $NR^6R^7$ wherein $R^6$ and $R^7$ each is lower alkyl. The compound of formula Vb in which $R^8$ or $R^9$ is $NR^{10}R^{11}$ wherein $R^{10}$ and $R^{11}$ each is benzyl or lower alkyl is subjected to acidic conditions, e.g. by dissolving the latter compound in a solution of acetone, diethyl ether and hydrochloric acid, and allowing the solution to stand at 20 to 30°C for one to three hours, to obtain the corresponding compound of formula VI in which $R^8$ or $R^9$ is $NR^{10}R^{11}$ wherein $R^{10}$ and $R^{11}$ are as defined herein. Reaction of the compound of formula VI with an excess of hydroxylamine hydrochloride in a solution of ethanol and pyridine at 20 to 30°C for 20 to 40 hours gives the corresponding compound of formula VII in which $R^8$ or $R^9$ is $NR^{10}R^{11}$ wherein $R^{10}$ and $R^{11}$ are as defined herein. Semmler-Wolff aromatization of the latter compound affords the corresponding compound of formula VIII in which $R^8$ or $R^9$ is $NR^{10}R^{11}$ wherein $R^{10}$ and $R^{11}$ are as defined herein. The aromatization can be achieved by reacting the compound of formula VII with about two molar equivalents of acetic anhydride in acetic acid for about 20 minutes, adding hydrogen bromide and stirring the resultant solution at about 85°C for about two hours. Another preferred method of aromatization involves the reaction of the compound of formula VII with acetyl chloride for about one hour at about 20 to 30°C. Hydrolysis of the compound of formula VIII, e.g. with hydrochloric acid at about 100°C for about one to five hours, gives the corresponding diamine of formula II in which $R^8$ or $R^9$ is $NR^{10}R^{11}$ wherein $R^{10}$ and $R^{11}$ are as defined herein.

Reaction scheme 2 illustrates a method for converting the compound of formula II to the compounds of formula I in which $R^2$ and $R^3$ are hydrogen.

REACTION SCHEME 2

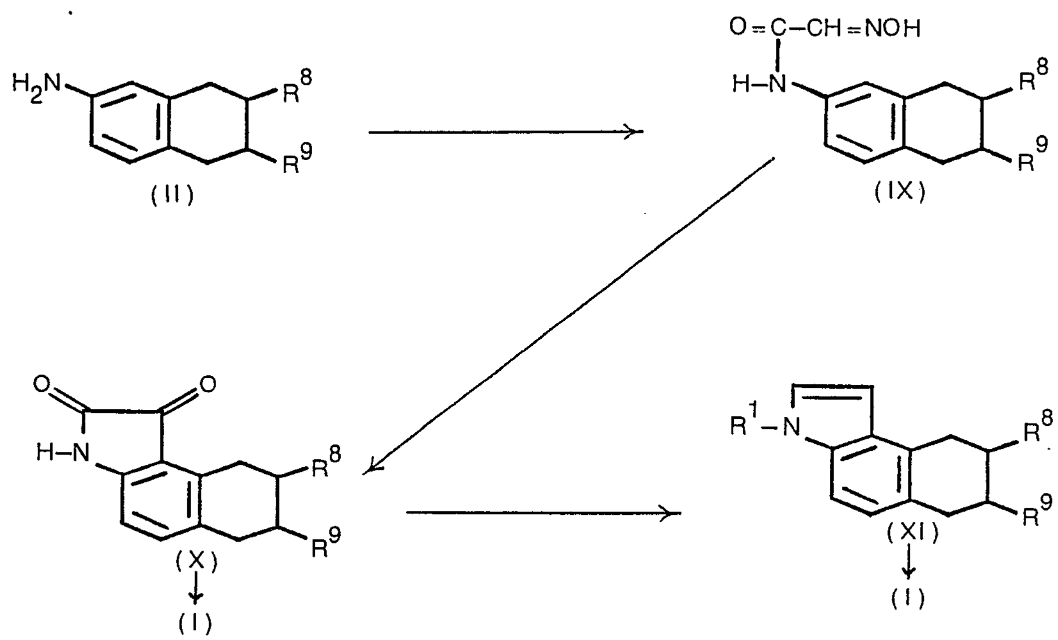


With reference to reaction scheme 2, a compound of formula II is reacted with hydroxylamine and a tribromo- or trichloro-acetaldehyde or corresponding hydrate in the presence of acid to give a compound of formula IX. For example a solution of the compound of formula II, about three to four molar equivalents of hydroxylamine hydrochloride and about six to seven molar equivalents of sodium sulfate in about 5 per cent hydrochloric acid is heated to about 100°C and a solution of about 13 molar equivalents of chloral hydrate in water is added. The resulting solution is maintained at about 100°C for about one to two hours to give the corresponding compound of formula IX in which $R^8$ or $R^9$ is $NR^{10}R^{11}$

wherein $R^{10}$ and $R^{11}$ are as defined herein. Cyclization of the latter compound according to the procedure of the Sandmeyer Isonitrosoacetanilide Isatin reaction (see Merck Index — 9th Edition ONR-79 and references therein), e.g. with concentrated sulphuric acid or polyphosphoric at about 0 to 80°C for 0.5 to two hours gives the corresponding compound of formula X in which $R^8$ or $R^9$ is $NR^{10}R^{11}$ wherein $R^{10}$ and $R^{11}$ are as defined herein.

Reduction of the compound of formula X in which $R^8$ or $R^9$ is $NR^{10}R^{11}$ wherein $R^{10}$ and $R^{11}$ are lower alkyl with a complex metal hydride gives the corresponding compound of formula I in which $R^1$, $R^2$ and $R^3$ are hydrogen, and $R^4$ or $R^5$ is $NR^6R^7$ wherein $R^6$ and $R^7$ each is lower alkyl. This reduction can be achieved conveniently by reacting the compound of formula X with about ten molar equivalents of lithium aluminium hydride in an inert organic solvent, for example, tetrahydrofuran or diethyl ether, at 20 to 30°C for 0.5 to ten hours to give the corresponding compound of formula I in which $R^1$, $R^2$ and $R^3$ are hydrogen, and $R^4$ or $R^5$ is $NR^6R^7$ wherein $R^6$ and $R^7$ each is lower alkyl. Alkylation of the latter compound of formula I gives the corresponding compound of formula I in which $R^1$ is lower alkyl, $R^2$ and $R^3$ are hydrogen, and $R^4$ or $R^5$ is $NR^6R^7$ wherein $R^6$ and $R^7$ each is lower alkyl. A convenient method of alkylation is the reaction of the compound of formula I with about one and a half to two molar equivalents of sodium amide (prepared from sodium in liquid ammonia containing ferric nitrate) in a mixture of diethyl ether and liquid ammonia to generate the corresponding anion. Reaction of this anion in the latter solvent system with a lower alkyl iodide, chloride or bromide for about one hour gives the corresponding compound of formula I in which $R^1$, $R^2$, $R^3$, $R^4$ and $R^5$ are as defined immediately above.

Similarly, reduction of the compound of formula X in which $R^8$ or $R^9$ is $NR^{10}R^{11}$ wherein $R^{10}$ is benzyl or lower alkyl with the complex metal hydride gives the corresponding compound of formula XI in which $R^1$ is hydrogen, and $R^8$ or $R^9$ is $NR^{10}R^{11}$ wherein $R^{10}$ is benzyl and $R^{11}$ is benzyl or lower alkyl. Alkylation of the latter compound, in the same manner as described above, gives the corresponding compound of formula XI in which $R^1$ is lower alkyl, and $R^8$ or $R^9$ is $NR^{10}R^{11}$ wherein $R^{10}$ is benzyl and $R^{11}$ is benzyl or lower alkyl. Hydrogenation of the compound of formula XI in which $R^1$ is hydrogen or lower alkyl, and $R^8$ or $R^9$ is $NR^{10}R^{11}$ wherein $R^{10}$ is benzyl and $R^{11}$ is benzyl or lower alkyl in the presence of a noble metal hydrogenation catalyst, for example, platinum on carbon, palladium on carbon or platinum oxide, in an inert solvent, for example, methanol or ethanol, gives the corresponding compound of formula I in which $R^1$ is hydrogen or lower alkyl, $R^2$ and $R^3$ are hydrogen, and $R^4$ or $R^5$ is $NR^6R^7$ wherein $R^6$ is hydrogen and $R^7$ is hydrogen or lower alkyl.

Reaction scheme 3 illustrates a method for converting the compound of formula II to the compounds of formula I in which $R^2$ and/or $R^3$ are lower alkyl.

REACTION SCHEME 3

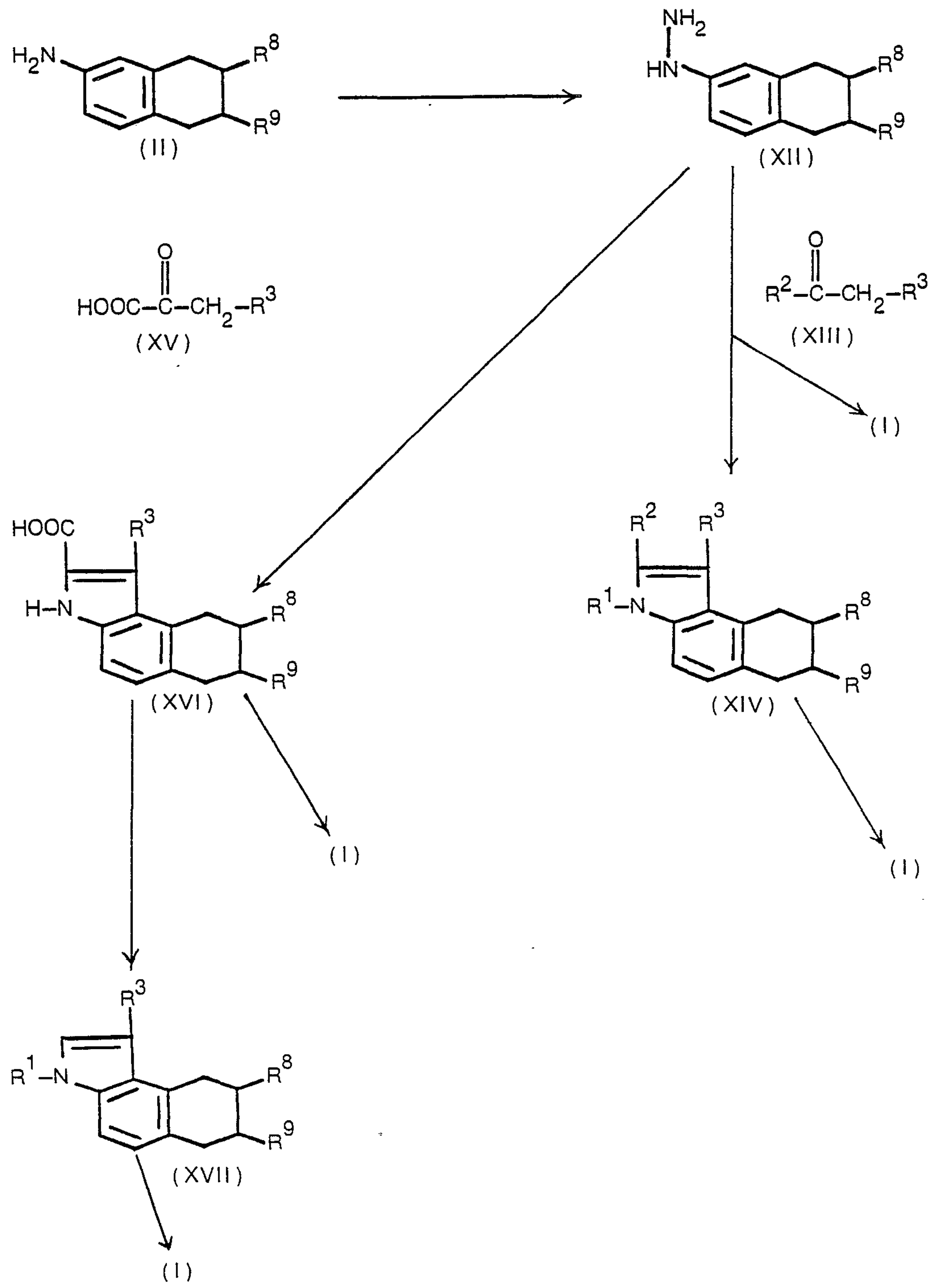

With reference to reaction scheme 3, the diamine of formula II in which $R^8$ or $R^9$ is $NR^{10}R^{11}$ wherein $R^{10}$ and $R^{11}$ are as defined herein is converted to the corresponding hydrazide of formula XII in which $R^8$ or $R^9$ is $NR^{10}R^{11}$ wherein $R^{10}$ and $R^{11}$ are as defined herein. For this conversion, a solution of the compound of formula II and about an equimolar amount of sodium nitrite in hydrochloric acid is maintained at about 0°C for one to five hours. A solution of about two and half molar equivalents of stannous chloride in hydrochloric acid is added at about −5 to −15°C. The mixture is maintained at this temperature for about one to five hours. Thereafter the corresponding compound of formula XII is isolated.

Condensation of the hydrazide of formula XII in which $R^8$ or $R^9$ is $NR^{10}R^{11}$ wherein $R^{10}$ and $R^{11}$ each is lower alkyl with a ketone of formula XIII in which $R^2$ is lower alkyl and $R^3$ is hydrogen or lower alkyl according to the Fischer indole synthesis gives the corresponding compound of formula I in which $R^1$ is hydrogen, $R^2$ is lower alkyl, $R^3$ is hydrogen or lower alkyl, and $R^4$ or $R^5$ is $NR^6R^7$ wherein $R^6$ and $R^7$ each is lower alkyl. The indole synthesis is achieved by maintaining a solution of about equal molar quantities of the compounds of formulae XII and XIII in acetic acid at about 100 to 120°C for about three to ten hours. Similarly, condensation of the compound of formula XII in which $R^8$ or $R^9$ is $NR^{10}R^{11}$ wherein $R^{10}$ is benzyl and $R^{11}$ is benzyl or lower alkyl with the ketone of formula XIII in which $R^2$ is lower alkyl and $R^3$ is hydrogen or lower alkyl according to the Fischer indole synthesis gives the corresponding compound of formula XIV in which $R^1$ is hydrogen, $R^2$ is lower alkyl, $R^3$ is hydrogen or lower alkyl, $R^8$ or $R^9$ is $NR^{10}R^{11}$ wherein $R^{10}$ is benzyl and $R^{11}$ is benzyl or lower alkyl. Alkylation of the latter compound, in the same manner as described above, gives the corresponding compound of formula XIV in which $R^1$ is lower alkyl and $R^2$, $R^3$, $R^8$ and $R^9$ are as defined immediately above. Hydrogenation of the compound of formula XIV in which $R^1$ is hydrogen or lower alkyl and $R^2$, $R^3$, $R^8$ and $R^9$ are as defined immediately above, in the same manner as described above, gives the corresponding compound of formula I in which $R^1$ and $R^3$ each is hydrogen or lower alkyl and $R^2$ is lower alkyl and $R^4$ or $R^5$ is $NR^6R^7$ wherein $R^6$ is hydrogen and $R^7$ is hydrogen or lower alkyl.

A Fischer indole condensation of the compound of formula XII in which $R^8$ or $R^9$ is $NR^{10}R^{11}$ wherein $R^{10}$ and $R^{11}$ are as defined herein with a keto-acid of formula XV in which $R^3$ is lower alkyl, in the same manner as described above, gives the corresponding compound of formula XVI in which $R^3$ is lower alkyl and $R^8$ or $R^9$ is $NR^{10}R^{11}$ wherein $R^{10}$ and $R^{11}$ are as defined herein. Decarboxylation of the compound of formula XVI in which $R^8$ or $R^9$ is $NR^{10}R^{11}$ wherein $R^{10}$ and $R^{11}$ each is lower alkyl, preferably with three to ten normal sulfuric acid at 50 to 100°C, yields the corresponding compound of formula I in which $R^1$ and $R^2$ are hydrogen, $R^3$ is lower alkyl, and $R^4$ or $R^5$ is $NR^6R^7$ wherein $R^6$ and $R^7$ each is lower alkyl. Similarly, decarboxylation of the compound of formula XVI in which $R^3$ is lower alkyl, and $R^8$ or $R^9$ is $NR^{10}R^{11}$ wherein $R^{10}$ is benzyl and $R^{11}$ is benzyl or lower alkyl gives the corresponding compound of formula XVII in which $R^1$ is hydrogen and $R^3$, $R^8$ and $R^9$ are as defined immediately above. The latter compound can be alkylated, in the same manner as described above, to give the corresponding compound of formula XVII in which $R^1$ is lower alkyl and $R^3$, $R^8$ and $R^9$ are as defined immediately above. Hydrogenation of the compound of formula XVII in which $R^1$ is hydrogen or lower alkyl and $R^3$, $R^8$ and $R^9$ are as defined immediately above, in the same manner as described above, gives the corresponding compound of formula I in which $R^1$ is hydrogen or lower alkyl, $R^2$ is hydrogen, $R^3$ is lower alkyl, and $R^4$ or $R^5$ is $NR^6R^7$ wherein $R^6$ is hydrogen and $R^7$ is hydrogen or lower alkyl.

If desired, the compounds of formula I in which $R^1$ is hydrogen, $R^2$ and $R^3$ each is hydrogen or lower alkyl, and $R^4$ or $R^5$ is $NR^6R^7$ wherein $R^6$ and $R^7$ each is lower alkyl can be alkylated, in the same manner as described above, to provide the corresponding compound of formula I in which $R^1$ is lower alkyl, $R^2$ and $R^3$ each is hydrogen or lower alkyl, and $R^4$ or $R^5$ is $NR^6R^7$ wherein $R^6$ and $R^7$ each is lower alkyl.

The following examples further illustrate this invention.

## Example 1

Propylamine hydrochloride (169 g, 1.8 mol) was dissolved in methanol (370 ml) and 6-methoxy-2-tetralone (50 g, 0.28 mol) was added. Thereafter, sodium cyanoborohydride (44.2 g, 0.7 mol) was added portionwise with cooling. During the latter addition, the temperature of the reaction mixture was kept at 20°C to minimize foaming. The resulting suspension was stirred at room temperature (25°C) for 18 hr. Excess hydrochloric acid (1:1) was added and the white suspension was cooled in ice. The solid was collected on a filter and washed with methanol (3 x 200 ml) to give 92 g of crude product. The crude product was treated with 10% (w/v) sodium hydroxide and extracted with benzene. The organic extract was dried and evaporated to give a pale yellow oil (31.93 g) of N-propyl-6-methoxy-1,2,3,4-tetrahydro-2-naphthylamine, NMR ($CDCl_3$) $\delta$ 0.90 (t, 3H), 1.15 (s, 1H), 1.50 (m, 2H), 3.70 (s, 3H), 6.55 (s, 1H), 6.60 (d, 1H) and 6.90 (d, 1H).

Sodium borohydride (26.85 g, 0.71 mol) was added in small portions to propionic acid (172.2 ml, 2.3 moles) in dry benzene (650 ml), maintaining the temperature below 20°C. When hydrogen evolution ceased, a solution of N-propyl-6-methoxy-1,2,3,4-tetrahydro-2-naphthylamine (30.93 g, 0.141 mol) in benzene (150 ml) was added and the resulting mixture was refluxed for 3 hr. The cooled mixture was shaken with excess 10% (w/v) sodium hydroxide solution solution. The organic layer was separated, dried over $MgSO_4$ and evaporated to give a brown oil of N,N-dipropyl-6-methoxy-1,2,3,4-tetrahydro-2-naphthylamine, NMR ($CDCl_3$) $\delta$ 0.85 (t, 6H), 1.45 (m, 6H), 2.45 (t, 4H), 2.7 (m, 5H), 3.70 (s, 3H) and 6.7 (m, 3H).

A solution of N,N-dipropyl-6-methoxy-1,2,3,4-tetrahydro-2-naphthylamine (17.6 g, 0.067 mole) in tetrahydrofuran (188 ml) and isopropanol (188 ml) was added with stirring to liquid ammonia (500 ml) cooled by a dry ice-isopropanol bath. Sodium (29.4 g, 1.3 g-atom) was added in small pieces over 0.5 hr. After the blue colour disappeared (ca. 2 hr), methanol (120 ml) was added and the ammonia was allowed to evaporate after removal of the cooling bath. The residue was diluted with water (1000

ml) and extracted three times with diethyl ether. The ether extracts were dried ($MgSO_4$) and concentrated, giving a yellow oil (15.8 g) of 1,2,3,4,5,8-hexahydro-6-methoxy-N,N-dipropyl-2-naphthalenamine, NMR ($CDCl_3$) $\delta$ 0.85 (t, 6H), s, 3H) and 4.6 (m, 1H).

A solution of 1,2,3,4,5,8-hexahydro-6-methoxy-N,N-dipropyl-2-naphthalenamine (32.5 g, 0.12 mole) in a mixture of acetone (542 ml), water (72 ml) and diethyl ether saturated with hydrogen chloride (270 ml) was stirred at room temperature for 3 hr, basified with sodium carbonate and extracted with diethyl ether. The ether extracts were dried ($MgSO_4$) and concentrated giving a mixture of 6-(N,N-dipropylamino)-2,3,4,4a,5,6,7,8-octahydronaphthalen-2-one and 6-(N,N-dipropylamino)-1,2,3,4,5,6,7,8-octahydronaphthalen-2-one as a brown oil (22.3 g): NMR ($CDCl_3$) $\delta$ 0.85 (t, 6H) and 5.75 (s, 1H) and IR ($CHCl_3$) 1705 1660 and 1615 $cm^{-1}$.

A solution of the latter mixture (22.0 g, 0.088 ml) and hydroxylamine hydrochloride (21.7 g) in ethanol (208 ml) and pyridine (197 ml) was stirred at room temperature for 18 hr. The mixture was concentrated, dissolved in water, basified with excess sodium bicarbonate and extracted into chloroform. The extract was dried ($MgSO_4$) and concentrated to give a red oil (22.7 g) of 6-(N,N-dipropylamino)-2,3,4,4a,5,6,7,8-octahydronaphthalen-2-oxime and 6-(N,N-dipropylamino)-1,2,3,4,5,6,7,8-octahydronaphthalen-2-oxime; NMR ($CDCl_3$) $\delta$ 0.95 (t, 6H), 5.9 and 6.6 (singlets, 1H).

Acetic anhydride (15.7 ml, 0.166 mole) was added to a stired solution of the latter mixture of oximes (22.1 g, 0.083 mole) in acetic acid (175 ml). The mixture was stirred at 35°C for 30 min and anhydrous gaseous hydrogen bromide was slowly passed through the solution until a temperature of 78°C was attained. The flow of hydrogen bromide was stopped and the dark solution was stirred at 85°C for 2 hr. After cooling, the solution was concentrated under reduced pressure and a sodium carbonate solution (~200 ml) was added to the reaction mixture until the pH became basic. The product was extracted with ethyl acetate. The organic extract was dried over $MgSO_4$, treated with charcoal, filtered through diatomaceous earth and evaporated to dryness to afford a brown oil (18.2 g) of N-[6-(N,N-dipropylamino)-5,6,7,8-tetrahydro-2-naphthalenyl]acetamide. A sample (2.44 g) was treated with acetic anhydride (8.5 ml) and pyridine (7.3 ml) at room temperature for 18 hr. The reaction mixture was evaporated to dryness and partitioned between water and ethyl acetate. The aqueous phase was basified with potassium carbonate and extracted with ethyl acetate to afford a·solid compound (1.62 g) which crystallized out of benzene and hexane to give a brown solid (723 mg) of N-[6-(N,N-dipropylamino)-5,6,7,8-tetrahydro-2-naphthalenyl]acetamide, mp 119—121°C and NMR ($CDCl_3$) $\delta$ 0.85 (t, 6H), 1.4 (m, 6H), 2.10 (s, 3H) and 6.8 to 7.3 (m, 3H).

A mixture of N-[6-(dipropylamino)-5,6,7,8-tetrahydro-2-naphthalenyl]acetamide (13.78 g, 0.057 mole) in 2N hydrochloric acid (37 ml) was refluxed for 2 hr. After cooling, the solution was basified with 1N sodium hydroxide and extracted four times with benzene. The organic extracts were dried ($MgSO_4$) and concentrated to afford a red oil (9.81 g) of 1,2,3,4-tetrahydro-$N^2$,$N^2$-dipropyl-2,6-naphthalenediamine: NMR ($CDCl_3$) $\delta$ 0.85 (t, 6H), 1.0 to 3.0 (m, 15H), 5.3 (b, 2H) and 6.3 to 7.5 (m, 3H).

1,2,3,4-Tetrahydro-$N^2$,$N^2$-dipropyl-2,6-naphthalenediamine (8.81 g, 0.035 mole) was dissolved in diethyl ether and a solution of hydrogen chloride in diethyl ether was added. The solution was evaporated and the residue was dissolved in water (100 ml). Hydroxylamine hydrochloride (8.27 g, 0.117 mole) and anhydrous sodium sulfate (34.14 g, 0.234 mole) was added. The mixture was brought to a boil, and immediately, a boiling solution of chloral hydrate (7.34 g) in water (108 ml) was added. The combined mixture was boiled for one hr, cooled and diluted with ammonium hydroxide (20 ml of conc. ammonium hydroxide in 200 ml water). The mixture was extracted with ethyl acetate (3×) and the ethyl extracts were combined, dried and evaporated to dryness to afford a brown solid (6.0 g) which was chromatographed through a column of silica gel (180 g) using chloroform and 1 to 10% methanol-chloroform (v/v). The appropriate eluates were evaporated and the residue was crystallized from dichloromethane-hexane to give N-[6-(dipropylamino)-5,6,7,8-tetrahydro-2-naphthalenyl]-2-(hydroxyimino)acetamide, m.p. 110—112°C; NMR ($CDCl_3$) $\delta$ 0.90 (t, 6H), 1.51 (m, 6H), 6.95 (s, 2H), 7.20 (s, 1H), 7.50 (s, 1H), 8.0 (s, 1H), and 10.3 (b, 1H); and Anal. Calcd. for $C_{18}H_{27}N_3O_2$: C, 68.10% H, 8.57% N, 13.23% and Found: C, 67.81% H, 8.50% N, 13.20%.

N-[6-(Dipropylamino)-5,6,7,8-tetrahydro-2-naphthalenyl]-2-(hydroxyimino)acetamide (1.49 g, 4.7 mmole) was added at 0°C to a rapidly stirred solution of concentrated sulfuric acid (24 ml). After stirring for one hr at 0°C, the reaction mixture was warmed to room temperature for 2 hr and poured onto ice-water (200 ml). The mixture was basified with concentrated ammonium hydroxide (80 ml) and extracted with ethyl acetate. The organic extracts were dried ($MgSO_4$) and evaporated to dryness to afford a red foam (1.49 g) which was chromatographed through silica gel using chloroform and 1 to 10%; methanol-chloroform (v/v). Evaporation of the eluates afforded 6,7,8,9-tetrahydro-7-(dipropylamino)-3H-benz[e]indole-1,2-dione (1.07 g) which was crystallized from diethyl ether-hexane; m.p. 83—86°C; NMR ($CDCl_3$) $\delta$ 0.85 (t, 6H), 1.45 (m, 6H), 1.7—3.6 (m, 9H), 6.60 (d, 1H) and 7.15 (d, 1H); and Anal. Calcd. for $C_{18}H_{24}N_2O_2$. 5.29% $H_2O$: C, 68.16% H, 8.21% N, 8.83% and Found: C, 68.28% H, 8.11% N, 8.99%.

To a cooled suspension of lithium aluminum hydride (3.2 g, 84.3 mmoles) in tetrahydrofuran (215 ml) under nitrogen was added dropwise a solution of 6,7,8,9-tetrahydro-7-(dipropylamino)-3H-benz[e]-indole-1,2,-dione (2.5 g, 8.32 mmole) in tetrahydrofuran (50 ml) and the mixture was stirred at room temperature for 2 hr. The excess of lithium aluminum hydride was destroyed at 0°C by careful addition

of a 10% (v/v) mixture of water in tetrahydrofuran (50 ml). The inorganic salts were filtered off through diatomaceous earth and washed with tetrahydrofuran. The filtrate was concentrated and water was added. The product was extracted in diethyl ether. The organic extracts were dried ($MgSO_4$) and concentrated to dryness to afford a blue oil (1.75 g) which was chromatographed through a column of silica gel (110 g) using 3% methanol-diethyl ether (v/v). Evaporation of the eluates gave 0.518 g of 6,7,8,9-tetrahydro-N,N-dipropyl-3H-benz[e]indol-7-amine, which was crystallized from methanol-water to give crystals (0.344 g) of the latter compound of formula I: mp 101—104°C; NMR ($CDCl_3$) $\delta$ 0.9 (t, 6H), 1.50 (m, 6H), 2.05—3.05 (m, 9H), 6.45 (m, 1H), 7.0 (m, 3H) and 8.1 (b, 1H): and Anal. Calcd. for $C_{18}H_{26}N_2$: C, 79.94% H, 9.69% N, 10.36% and Found: C, 80.07% H, 9.76% N, 10.31%.

Example 2

7-Methoxy-2-tetralone (35.1 g, 0.20 mole) was dissolved in dry benzene (1000 ml) and put in a 2000 ml three neck-flask. To this solution was added dipropylamine (109 ml, 0.80 mole) and p-toluenesulfonic acid monohydrate (34 g, 0.2 mole). This mixture was refluxed under nitrogen with continuous water removal for 48 hr. The benzene was replaced by ethanol (600 ml, anhydrous) and the solution was hydrogenated in a 2 liter pressure bottle over platinum oxide (1.0 g) under 2 atmospheres of pressure at room temperature for 2.5 hr. The solution was filtered through cellulose powder and concentrated. Benzene was added to the filtrate. The organic solution was basified with 10% sodium hydroxide, washed with water and extracted with 5% hydrochloric acid. The aqueous solution was basified with 10% sodium hydroxide and extracted with diethyl ether. The organic extract was dried and evaporated to give a brown liquid (36.5 g) of N,N-dipropyl-7-methoxy-1,2,3,4-tetrahydro-2-naphthylamine. A sample was purified by chromatography through a column of silica gel using 2% methanol-chloroform mixture as eluant. The hydrochloride salt was prepared and crystallized from methanol and diethyl either: mp 152—157°C, and Anal. Calcd. for $C_{17}H_{28}NOCl$. $H_2O$: C, 66.53% H, 9.45% N, 4.56% and Found: C, 66.39% H, 9.40% N, 4.39%.

The latter procedure was repeated with benzylamine replacing dipropylamine to obtain N-benzyl-7-methoxy-1,2,3,4-tetrahydro-2-naphthylamine hydrochloride: mp 252—255°C (crystallized from methanol-diethyl ether) and Anal. Calcd. for $C_{18}H_{22}ClNO$: C, 71.15% H, 7.30% N, 4.61% and Found: C, 70.95% H, 7.24; N, 4.49%.

A solution of N-benzyl-7-methoxy-1,2,3,4-tetrahydro-2-naphthylamine (71 g, 0.255 mole) in anhydrous ethanol (600 ml) in a 2 litre pressure bottle (Parr Shaker) was hydrogenated over 10% palladium on charcoal (28 g) under 2 atmospheres pressure at room temperature for 7 hr. The mixture was filtered and the filtrate was evaporated. A sample of the residue was treated with a solution of hydrogen chloride in diethyl ether and the precipitate was crystallized from methanol-diethyl ether to give 7-methoxy-1,2,3,4-tetrahydronaphthalen-2-amine hydrochloride: mp 213—215°C, and Anal. Calcd. for $C_{11}H_{16}ClNO$: C, 61.82% H, 7.54% N, 6.55% and Found: C, 61.53% H, 7.45% N, 6.45%.

A mixture of 7-methoxy-1,2,3,4-tetrahydronaphthalen-2-amine (35.9 g, 0.203 mole), 98% formic acid (49 ml, 1.30 mole) and 37% formaldehyde (36.9 ml, 0.451 mole) was placed in a 500 ml flask and heated on a steam bath for 1.5 h. The reaction mixture was then concentrated under reduced pressure, neutralized with 10% sodium hydroxide (150 ml) and extracted with diethyl ether. The ether extracts were dried and evaporated to give a red oil (41.5 g). To a solution of HCl in diethyl ether (110 ml) in a 3 liter flask, under nitrogen at 0°C was added dropwise a solution of this oil in diethyl ether (300 ml). After stirring mechanically for 4 hr, the pink solid was filtered, washed with diethyl ether and converted back to the pure free base (38.6 g) with 10% sodium hydroxide. A sample of hydrochloride salt was crystallized from methanol and diethyl ether to give N,N-dimethyl-7-methoxy-1,2,3,4-tetrahydro-naphthalen-2-amine hydrochloride: mp 222—225°C; NMR ($CDCl_3$) of free base: $\delta$ 2.35 (s, 6H), 1.65 (m, 2H), 2.7 (m, 5H), 3.7 (s, 3H) and 6.75 (m, 3H).

A solution of N,N-dipropyl-7-methoxy-1,2,3,4-tetrahydro-2-naphthylamine (17.3 g, 0.066 mole) in tetrahydrofuran (200 ml) and isopropanol (200 ml) was added with stirring to liquid ammonia (500 ml) cooled by a dry ice-isopropanol bath. Sodium (30 g, 1.3 g-atoms) was added in small pieces over 0.5 hr. After the blue colour disappeared (ca. 2 hr), methanol (130 ml) was added and the ammonia was allowed to evaporate after removal of the cooling bath. The residue was diluted with water (1000 ml) and extracted three times with diethyl ether. The ether extracts were dried ($MgSO_4$) and concentrated, giving a yellow solid (13.6 g). The solid was recrystallized from a mixture of acetone and water to give a white solid (12.7 g) of 1,2,3,4,5,8-hexahydro-7-methoxy-N,N-dipropyl-2-naphthalen-amine: mp 47—51°C and Anal. Calcd. for $C_{17}H_{29}NO$: C, 77.51% H, 11.09% N, 5.31% and Found: C, 77.21% H, 11.07% N, 5.37%.

The latter procedure was repeated with N,N-dimethyl-7-methoxy-1,2,3,4-tetrahydronaphthalen-2-amine replacing N,N-dipropyl-7-methoxy-1,2,3,4-tetrahydro-2-naphthylamine to obtain an oil of N,N-dimethyl-1,2,3,4,5,8-hexahydro-7-methoxy-2-naphthalenamine; NMR ($CDCl_3$) $\delta$ 1.5 (m, 2H), 2.0 (m, 4H), 2.3 (s, 6H), 2.6 (s, 4H), 2.9 (m, 1H), 3.55 (s, 3H) and 4.6 (s, 1H).

A solution of 1,2,3,4,5,8-hexahydro-7-methoxy-N,N-dipropyl-2-naphthalenamine (24.8 g, 0.094 mole) in a mixture of acetone (415 ml), water (55 ml) and diethyl ether saturated with hydrogen chloride (207 ml) was stirred at room temperature for 1.5 hr; basified with sodium carbonate and extracted with diethyl ether. The ether extracts were dried ($MgSO_4$) and concentrated giving a mixture

of 7-(N,N-dipropylamino)-2,3,4,4a,5,6,7,8-octahydronaphthalen-2-one and 7-(N,N-dipropylamino)-1,2,3,4,5,6,7,8-octahydronaphthalen-2-one as a brown oil (20.5 g): NMR ($CDCl_3$) $\delta$ 0.80 (t, 6H) and 5.8 (s, 1H) and IR ($CHCl_3$) 1710, 1660 and 1620 $cm^{-1}$.

The latter procedure was repeated with N,N-dimethyl-1,2,3,4,5,8-hexahydro-7-methoxy-2-naphthalenamine replacing 1,2,3,4,5,8-hexahydro-7-methoxy-N,N-dipropyl-2-naphthalenamine to obtain an oil of 7-(N,N-dimethylamino)-2,3,4,4a,5,6,7,8-octahydronaphthalen-2-one: NMR ($CDCl_3$) $\delta$ 2.25 (s, 6H) 5.65—6.10 (m, 1H); and IR ($CHCl_3$) 1665 $cm^{-1}$.

A solution of the mixture of 7-(N,N-dipropylamino)-2,3,4,4a,5,6,7,8-octahydronaphthalen-2-one and 7-(N,N-dipropylamino)-1,2,3,4,5,6,7,8-octahydronaphthalene-2-one (18.4 g, 0.074 mole) and hydroxylamine hydrochloride (22 g, 0.32 mole) in ethanol (211 ml) and pyridine (197 ml) was stirred at room temperature overnight. The mixture was concentrated, dissolved in water, basified with excess sodium bicarbonate and extracted into chloroform. The extract was dried ($MgSO_4$) and concentrated to give a red oil (18.9 g) of 7-(N,N-dipropylamino)-2,3,4,4a,5,6,7,8-octahydronaphthalen-2-oxime and 7-(N,N-dipropylamino)-1,2,3,4,5,6,7,8-octahydronaphthalen-2-oxime; NMR ($CDCl_3$) $\delta$ 0.95 (t, 6H), 5.9 and 6.6 (singlets, 1H) and 8.5 (br, 1H).

The latter procedure was repeated with 7-(N,N-dimethylamino)-2,3,4,4a,5,6,7,8-octahydronaphthalen-2-one replacing the mixture of octahydronaphthalen-2-ones to obtain 7-(N,N-dimethylamino)-2,3,4,4a,5,6,7,8-octahydronaphthalen-2-oxime hydrochloride: mp 211—213°C; Anal. Calcd. for $C_{12}H_{21}ClN_2O$: C, 58.68% H, 8.64% N, 11.44% and Found: C, 58.74% H, 8.76% N, 11.30%; and NMR ($CDCl_3$) of free base: $\delta$ 2.25 (s, 6H), and 5.85 (s, 1H).

Acetic anhydride (15.2 ml, 0.15 mole) was added to a stirred solution of the mixture of 7-(N,N-dipropylamino)-2,3,4,4a,5,6,7,8-octahydronaphthalen-2-oxime and 7-(N,N-dipropylamino)-1,2,3,4,5,6,7,8-octahydronaphthalen-2-oxime (21 g, 0.080 mole) in acetic acid (170 ml). The mixture was stirred for 20 min and anhydrous gaseous hydrogen bromide was slowly passed through the solution until a temperature of 75°C was attained. The flow of hydrogen bromide was stopped and the dark solution was stirred at 85°C for 2 hr. The solution was concentrated under reduced pressure and poured into a sodium carbonate solution (400 ml). A yellow solid (9.6 g) precipitated, collected by filtration and crystallized from methanol to give N-[7-(N,N-dipropylamino)-5,6,7,8-tetrahydro-2-naphthalenyl]acetamide hydrobromide: mp 282—285°C. The basic filtrate was extracted with ethyl acetate. The organic extracts were dried ($MgSO_4$) and concentrated to afford a brown oil (9.3 g) which was chromatographed through silica gel using 5% methanol in chloroform as eluant. Evaporation of the eluates afforded 3.5 g of N-[7-(N,N-dipropylamino)-5,6,7,8-tetrahydro2-naphthalenyl]acetamide: NMR ($CDCl_3$) $\delta$ 0.85 (t, 6H), 1.5 (m, 6H), 2.1 (s, 3H), 2.45 (m, 4H), 2.72 (m, 5H) and 7.65 (m, 3H).

The latter procedure was repeated with 7-(N,N-dimethylamino)-2,3,4,4a,5,6,7,8-octahydronaphthalen-2-omime hydrochloride replacing the mixture of oximes to obtain N-[7-(N,N-dimethylamino)-5,6,7,8-tetrahydro-2-naphthalenyl]-acetamide: mp 89—91°C (triturated from hexane); NMR ($CDCl_3$) $\delta$ 2.10 (s, 3H), 2.35 (s, 6H) and 7.1 (m, 3H); and Anal. Calcd. for $C_{14}H_{20}N_2O$: C, 72.37% H, 8.67% N, 12.06% and Found: C, 72.30% H, 8.81% N, 11.99%.

A suspension of N-[7-(dipropylamino)-5,6,7,8-tetrahydro-2-naphthalenyl]-acetamide hydrobromide (3.0 g, 8.1 mmoles) in water was treated in 1N sodium hydroxide (100 ml) and the resulting free base was extracted in benzene. The solvent was evaporated and the residue was heated at reflux in 2N hydrochloric acid (37 ml) for 2 hr. After cooling, the solution was basified with 1N sodium hydroxide and extracted three times with benzene. The organic extracts were dried ($MgSO_4$) and concentrated to afford a red oil (1.76 g) of 1,2,3,4-tetrahydro-$N^2$,$N^2$-dipropyl-2,7-naphthalene-diamine: NMR ($CDCl_3$) $\delta$ 0.85 (t, 6H), 1.42 (m, 6H), 2.42 (t, 4H), 2.70 (m, 4H), 6.35 (m, 2H) and 6.80 (d, 1H). The di-(Z)-2-butenedioate salt was prepared in acetone and diethyl ether, and crystallized from methanol-diethyl ether: mp 124—126°C and Anal. Calcd. for $C_{16}H_{26}N_2.2C_4H_4O_2$: C, 60.23% H, 7.16% N, 5.85% and Found: C, 60.45% H, 7.21% N, 5.88%.

The latter procedure was repeated with N-[7-(N,N-dimethylamino)-5,6,7,8-tetrahydro-2-naphthalenyl]acetamide replacing N-[7-(dipropylamino)-5,6,7,8-tetrahydro-2-naphthalenyl]acetamide to obtain 1,2,3,4-tetrahydro-$N^2$,$N^2$-dimethyl-2,7-naphthalenediamine dihydrochloride: mp 278°C (crystallized from methanol-diethyl ether); Anal. Calcd. for $C_{12}H_{18}N_2.2HCl$: C, 54.75% H, 7.65% N, 10.64% and Found: C, 54.66% H, 7.54% N, 10.44%.

1,2,3,4-Tetrahydro-$N^2$,$N^2$-dipropyl-2,7-naphthalenediamine (3.1 g, 12.4 mmole) in water (37 ml) was carefully treated with 5% hydrochloric acid (30 ml), hydroxylamine hydrochloride (2.88 g, 41 mmoles) and anhydrous sodium sulfate (11.9 g, 82 mmole). The mixture was brought to a boil, and immediately, a boiling solution of chloral hydrate (2.56 g) in water (38 ml) was added. The combined mixture was boiled for one hr, cooled and diluted with ammonium hydroxide (8 ml of conc. ammonium hydroxide in 80 ml water). The precipitate was filtered and redissolved in ethyl acetate. The filtrate was extracted with ethyl acetate (3×) and the ethyl acetate solutions were combined, dried and evaporated to dryness to afford a brown solid (4.78 g) which was crystallized from dichloromethane to give N-[7-(dipropylamino)-5,6,7,8-tetrahydro-2-naphthalenyl]-2-(hydroxyimino)acetamide: mp 82—85°C; NMR ($CDCl_3$) $\delta$ 0.9 (t, 6H), 1.55 (m, 6H), 2.7 (m, 11H), 7.2 (m, 4H), 8.05 (s, 1H) and 11.0 (br, 1H); and Anal. Calcd. for $C_{18}H_{27}N_3O$: C, 68.10% H, 8.57% N, 13.23% and Found: C, 67.82% H, 8.63% N, 13.14%.

The latter procedure was repeated with 1,2,3,4-tetrahydro-$N^2$,$N^2$-dimethyl-2,7-naphthalene-

diamine dihydrochloride replacing 1,2,3,4-tetrahydro-$N^2$,$N^2$-dipropyl-2,7-naphthalenediamine to obtain N-[7-(dimethylamino)-5,6,7,8-tetrahydro-2-naphthalenyl]-2-(hydroxyimino)acetamide: NMR ($CDCl_3$) $\delta$ 1.70 (m, 2H), 2.25 (s, 6H), 2.65 (m, 5H), 6.95 (d, 1H), 7.30 (d, 1H), 7.35 (s, 1H), 7.60 (s, 1H) and 9.90 (s, 2H).

N-[7-(Dipropylamino)-5,6,7,8-tetrahydro-2-naphthalenyl]-2-(hydroxyimino)acetamide (3.66 g, 11.54 mmoles) was added at 0°C to a rapidly stirred solution of concentrated sulfuric acid (61 ml) and water (6.1 ml). After stirring for one hr at 0°C, the reaction mixture was warmed to 75°C for 0.5 hr, cooled to room temperature and poured onto cracked ice (600 ml). The mixture was basified with concentrated ammonium hydroxide (150 ml) and extracted with ethyl acetate. The organic extracts were dried ($MgSO_4$) and evaporated to dryness to afford a red foam (2.78 g). The foam was crystallized from diethyl ether-hexane to afford 8-(dipropylamino)-6,7,8,9-tetrahydro-3H-benz[e]-indole-1,2-dione: mp 124—126°C; NMR ($CDCl_3$) $\delta$ 6.6 (d, 1H), 7.15 (d, 1H) and 8.6 (br, 1H); and Anal. Calcd. for $C_{18}H_{24}N_2O_2$: C, 71.96% H, 8.05% N, 9.32% and Found: C, 71.56% H, 8.04% N, 9.22%.

The latter procedure was repeated with N-[7-(dimethylamino)-5,6,7,8-tetrahydro-2-naphthalenyl]-2-(hydroxyimino)acetamide replacing N-[7-(dipropylamino)-5,6,7,8-tetrahydro-2-naphthalenyl]-2-(hydroxyimino)acetamide to obtain 8-(dimethylamino)-6,7,8,9-tetrahydro-3H-benz[e]indole-1,2-dione: mp 181—184°C; NMR (DMSO-$d_6$) $\delta$ 2.20 (s, 6H), 6.55 (d, 2H) and 7.70 (d); and Anal. Calcd. for $C_{14}H_{16}N_2O_2$: C, 68.82% H, 6.60% H, 11.46% and Found: C, 66.65% H, 6.55% N, 11.34%.

To a cooled suspension of lithium aluminum hydride (1.5 g, 38.9 mmoles) in tetrahydrofuran (100 ml) under nitrogen was added dropwise a solution of 8-(dipropylamino)-6,7,8,9-tetrahydro-3H-benz[e]-indole-1,2-dione (1.17 g, 3.89 mmoles) in tetrahydrofuran (25 ml) and the mixture was stirred at room temperature for 2 hr. The excess of lithium aluminum hydride was destroyed by careful addition of a 10% mixture of water in tetrahydrofuran (50 ml). The inorganic salts were filtered off through diatomaceous earth and washed with tetrahydrofuran. The filtrate was concentrated and water was added. The product was extracted in diethyl ether. The organic extracts were dried ($MgSO_4$) and concentrated to dryness to afford a green oil (1.17 g) which was chromatographed through a column of silica gel using chloroform and 1%, 3% and 5% methanol-chloroform mixtures as eluant. Evaporation of the eluates gave 595 mg of 6,7,8,9-tetrahydro-N,N-dipropyl-3H-benz[e]indol-8-amine; NMR ($CDCl_3$) $\delta$ 0.80 (t, 6H), 1.1 to 3.5 (m, 15H), 6.45 (s, 1H), 7.0 (m, 3H) and 8.15 (br, 1H). A solution of the latter compound (440 mg) in diethyl ether was mixed with a solution of maleic acid (188 mg) in acetone. The precipitate was collected and crystallized from methanol-diethyl ether to give the maleate salt of 6,7,8,9-tetrahydro-N,N-dipropyl-3H-benz[e]indol-8-amine (325 mg): mp 164—165°C; NMR ($CDCl_3$) $\delta$ 1.0 (t, 6H), 1.9 (m, 6H), 3.1 (m, 9H), 6.25 (s, 2H), 6.4 (s, 1H), 6.82 (d, 1H), 7.2 (m, 2H), 8.8 (s, 1H) and 11.5 (br, 2H); and Anal. Calcd. for $C_{22}H_{30}N_2O_4$: C, 68.36% H, 7.82% N, 7.25% and Found: C, 68.18% H, 7.84% N, 7.05%.

The latter procedure was repeated with 8-(dimethylamino)-6,7,8,9-tetrahydro-3H-benz[e]indole-1,2-dione replacing 8-(dipropylamino)-6,7,8,9-tetrahydro-3H-benz[e]indole-1,2-dione to obtain 6,7,8,9-tetrahydro-N,N-dimethyl 3H-benz[e]indol-8-amine: mp 191—197°C NMR (DMSO-$d_6$) $\delta$ 2.3 (s, 6H), 2.5 (m, 7H), 6.35 (m, 1H), 6.60 (d, 2H), 7.10 (d), 7.20 (d, 1H) and 11.3 (s, 1H); and Anal. Calcd. for $C_{14}H_{18}N_2$: C, 78.46% H, 8.46% H, 13.07% and Found: C, 78.40% H, 8.44% N, 13.10%.

Example 3

6,7,8,9-Tetrahydro-N,N-dimethyl-3H-benz[e]indol-8-amine (380 mg, 1.8 mmole) was added slowly as a solid with stirring to a suspension of sodium amide, prepared from clean sodium (166 mg, 7.24 mmole) in liquid ammonia (13 ml) containing ferric nitrate (20 mg). The suspension was diluted with tetrahydrofuran (8 ml) before adding the starting material. After stirring for 15 min, methyl iodide (0.13 ml, 2.24 mmole) was added and the mixture was stirred for 6 hr. The ammonia was then allowed to evaporate overnight. The tetrahydrofuran was removed under reduced pressure and the residue was taken back in ethyl acetate, washed with water, dried with magnesium sulfate and evaporated to dryness. The yellow solid (276 mg) was dissolved in a minimum amount of chloroform and chromatographed on silica gel with a mixture of triethylamine-ethyl acetate-benzene (1:3:6 v/v) and increasing amount of methanol (2, 4, 6, 8, 10% v/v). Evaporation of the appropriate eluates gave 6,7,8,9-tetrahydro-N,N,3-trimethyl-3H-benz[e]indol-8-amine (133 mg): NMR ($CDCl_3$) $\delta$ 2.4 (s, 6H), 3.75 (s, 3H) and 6.8 (m, 4H). The latter compound was converted to the maleate salt which was crystallized from dichloromethane-diethyl ether: mp 178—179°C; and Anal. Calcd. for $C_{15}H_{20}N_2.C_4H_4O_4$: C, 66.25% H, 7.02% N, 8.13% and Found: C, 66.11% 7.18% N, 8.11%.

The latter procedure was repeated with 6,7,8,9-tetrahydro-N,N-dipropyl-3H-benz[e]indol-8-amine replacing 6,7,8,9-tetrahydro-N,N-dimethyl-3H-benz[e]indol-8-amine to obtain 6,7,8,9-tetrahydro-3-methyl-N,N-dipropyl-3H-benz[e]indol-8-amine which was converted to the hydrochloride: mp 131—134°C; NMR ($CDCl_3$) $\delta$ 1.05 (t, 6H), 2.0 (m, 6H), 3.1 (m, 9H), 3.75 (s, 3H), 7.8 (m, 4H) and 11.0 (br, 1H); and Anal. Calcd. for $C_{19}H_{28}N_2.HCl$: C, 71.11% H, 9.10% N, 8.73% and Found: C, 70.78% H, 8.95% N, 8.64%.

**Claims for the Contracting States: BE CH DE FR GB IT LI LU NL SE**

1. A compound of formula I

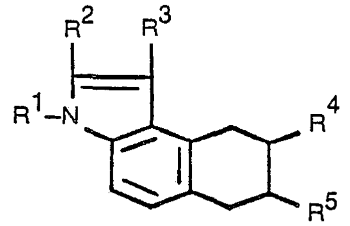

(I)

in which $R^1$, $R^2$ and $R^3$ each is hydrogen or lower alkyl having 1 to 5 carbon atoms, $R^4$ is $NR^6R^7$ wherein $R^6$ and $R^7$ each is hydrogen or lower alkyl having 1 to 5 carbon atoms and $R^5$ is hydrogen, or $R^4$ is hydrogen and $R^5$ is $NR^6R^7$ wherein $R^6$ and $R^7$ are as defined herein, or a therapeutically acceptable acid addition salt thereof.

2. A compound of Claim 1 in which $R^1$, $R^2$ and $R^3$ each is hydrogen or lower alkyl having 1 to 3 carbon atoms, $R^4$ is $NR^6R^7$ wherein $R^6$ and $R^7$ each is hydrogen or lower alkyl having 1 to 3 carbon atoms and $R^5$ is hydrogen, or $R^4$ is hydrogen and $R^5$ is $NR^6R^7$ wherein $R^6$ and $R^7$ each is hydrogen or lower alkyl having 1 to 3 carbon atoms, or a therapeutically acceptable acid addition salt thereof.

3. A compound as claimed in Claim 1 or Claim 2 in which $R^1$, $R^2$ and $R^3$ are hydrogen.

4. A compound as claimed in Claim 1 wherein $R^1$, $R^2$ and $R^3$ are hydrogen, $R^4$ is $NR^6R^7$ wherein $R^6$ and $R^7$ each is lower alkyl having 1 to 3 carbon atoms and $R^5$ is hydrogen, or $R^4$ is hydrogen and $R^5$ is $NR^6R^7$ wherein $R^6$ and $R^7$ each is lower alkyl having 1 to 3 carbon atoms, or a therapeutically acceptable acid addition salt thereof.

5. A compound as claimed in Claim 1, which is 6,7,8,9-tetrahydro-N,N-dipropyl-3H-benz[e]indol-7-amine; 6,7,8,9-tetrahydro-N,N-dipropyl-3H-benz[e]indol-8-amine; 6,7,8,9-tetrahydro-N,N-dimethyl-3H-benz[e]indol-8-amine; or a therapeutically acceptable acid addition salt thereof.

6. A compound of formula I which is 6,7,8,9-tetrahydro-N,N,3-trimethyl-3H-benz[e]indol-8-amine or 6,7,8,9-tetrahydro-3-methyl-N,N-dipropyl-3H-benz[e]indol-8-amine, or a therapeutically acceptable acid addition salt thereof.

7. A pharmaceutical composition comprising a compound as claimed in any one of Claims 1 to 6 or a therepeutically acceptable acid addition salt thereof, and a pharmaceutically acceptable carrier therefor.

8. A compound as claimed in any one of Claims 1 to 6 or a therapeutically acceptable acid addition salt thereof for use as a pharmaceutical.

9. A process for preparing a compound of formula I as defined in claim 1 or a therapeutically acceptable acid addition salt thereof which comprises one of the following:

(a) when a compound of formula I in which $R^1$, $R^2$ and $R^3$ are hydrogen, $R^4$ is $NR^6R^7$ wherein $R^6$ and $R^7$ each is lower alkyl having 1 to 5 carbon atoms and $R^5$ is hydrogen, or $R^4$ is hydrogen and $R^5$ is $NR^6R^7$ wherein $R^6$ and $R^7$ each is lower alkyl having 1 to 5 carbon atoms is required, reducing a corresponding compound of formula X

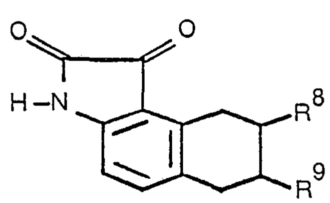

(X)

in which $R^8$ is $NR^{10}R^{11}$ wherein $R^{10}$ and $R^{11}$ each is lower alkyl and $R^9$ is hydrogen, or $R^8$ is hydrogen and $R^9$ is $NR^{10}R^{11}$ wherein $R^{10}$ and $R^{11}$ each is lower alkyl with a complex metal hydride;

(b) when a compound of formula I in which $R^1$ is hydrogen or lower alkyl, $R^2$ and $R^3$ are hydrogen, $R^4$ is $NR^6R^7$ wherein $R^6$ is hydrogen and $R^7$ is hydrogen or lower alkyl and $R^5$ is hydrogen, or $R^4$ is hydrogen and $R^5$ is $NR^6R^7$ wherein $R^6$ is hydrogen and $R^7$ is hydrogen or lower alkyl is required, hydrogenating a compound of formula XI

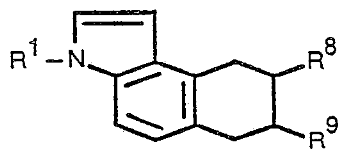

(XI)

in which $R^1$ is hydrogen or lower alkyl, $R^8$ is $NR^{10}R^{11}$ wherein $R^{10}$ is benzyl and $R^{11}$ is benzyl or lower alkyl and $R^9$ is hydrogen, or $R^8$ is hydrogen and $R^9$ is $NR^{10}R^{11}$ wherein $R^{10}$ is benzyl and $R^{11}$ is benzyl or lower alkyl;

(c) when a compound of formula I in which $R^1$ is hydrogen, $R^2$ is lower alkyl, $R^3$ is hydrogen or lower alkyl, $R^4$ is $NR^6R^7$ wherein $R^6$ and $R^7$ each is lower alkyl and $R^5$ is hydrogen, or $R^4$ is hydrogen and $R^5$ is $NR^6R^7$ wherein $R^6$ and $R^7$ each is lower alkyl is required, condensing a compound of formula XII

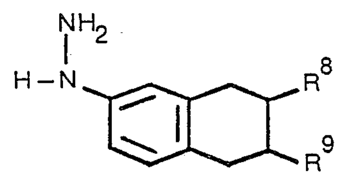

(XII)

in which $R^8$ is $NR^{10}R^{11}$ wherein $R^{10}$ and $R^{11}$ each is lower alkyl and $R^9$ is hydrogen, or $R^8$ is hydrogen and $R^9$ is $NR^{10}R^{11}$ wherein $R^{10}$ and $R^{11}$ each is lower alkyl with a ketone of the formula

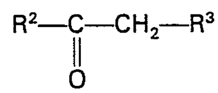

in which $R^2$ is lower alkyl and $R^3$ is hydrogen or lower alkyl according to the Fischer indole method;

(d) when a compound of formula I in which $R^1$ and $R^3$ is hydrogen or lower alkyl, $R^2$ is lower alkyl, $R^4$ is $NR^6R^7$ wherein $R^6$ is hydrogen and $R^7$ is hydrogen or lower alkyl and $R^5$ is hydrogen, or $R^4$ is hydrogen and $R^5$ is $NR^6R^7$ wherein $R^6$ is hydrogen and $R^7$ is hydrogen or lower alkyl is required, hydrogenating a corresponding compound of formula XIV

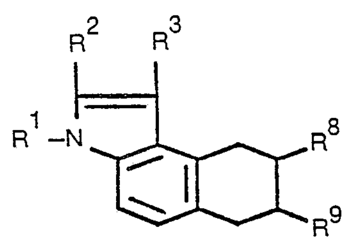

(XIV)

in which $R^1$ and $R^3$ each is hydrogen or lower alkyl, $R^2$ is lower alkyl, $R^8$ is $NR^{10}R^{11}$ wherein $R^{10}$ is benzyl and $R^{11}$ is benzyl or lower alkyl and $R^9$ is hydrogen, or $R^8$ is hydrogen and $R^9$ is $NR^{10}R^{11}$ wherein $R^{10}$ is benzyl and $R^{11}$ is benzyl or lower alkyl;

(e) when a compound of formula I in which $R^1$ and $R^2$ are hydrogen, $R^3$ is lower alkyl, $R^4$ is $NR^6R^7$ wherein $R^6$ and $R^7$ each is lower alkyl and $R^5$ is hydrogen, or $R^4$ is hydrogen and $R^5$ is $NR^6R^7$ wherein $R^6$ and $R^7$ each is lower alkyl is required, decarboxylating a corresponding compound of formula XVI

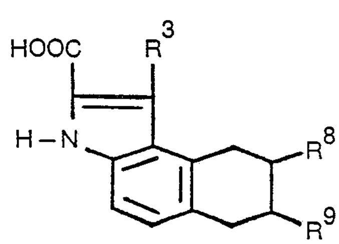

(XVI)

in which $R^3$ is lower alkyl, $R^8$ is $NR^{10}R^{11}$ wherein $R^{10}$ and $R^{11}$ each is lower alkyl and $R^9$ is hydrogen, or $R^8$ is hydrogen and $R^9$ is $NR^{10}R^{11}$ wherein $R^{10}$ and $R^{11}$ each is lower alkyl;

(f) when a compound of formula I in which $R^1$ is hydrogen or lower alkyl, $R^2$ is hydrogen, $R^3$ is lower alkyl, $R^4$ is $NR^6R^7$ wherein $R^6$ is hydrogen and $R^7$ is hydrogen or lower alkyl and $R^5$ is hydrogen, or $R^4$ is hydrogen and $R^5$ is $NR^6R^7$ wherein $R^6$ is hydrogen and $R^7$ is hydrogen or lower alkyl is required, hydrogenating a corresponding compound of formula XVII

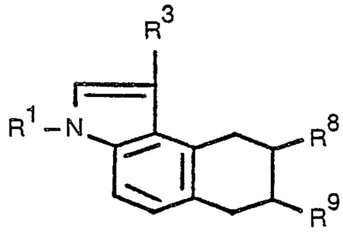

(XVII)

in which $R^1$ is hydrogen or lower alkyl, $R^3$ is lower alkyl, $R^8$ is $NR^{10}R^{11}$ wherein $R^{10}$ is benzyl and $R^{11}$ is benzyl or lower alkyl and $R^9$ is hydrogen, or $R^8$ is hydrogen and $R^9$ is $NR^{10}R^{11}$ wherein $R^{10}$ is benzyl and $R^{11}$ is benzyl or lower alkyl;

(g) when a compound of formula I in which $R^1$ is lower alkyl, $R^2$ and $R^3$ each is hydrogen or lower alkyl, $R^4$ is $NR^6R^7$ wherein $R^6$ and $R^7$ each is lower alkyl and $R^5$ is hydrogen, or $R^4$ is hydrogen and $R^5$ is $NR^6R^7$ wherein $R^6$ and $R^7$ each is lower alkyl, is required, alkylating the corresponding compound of formula I in which $R^1$ is hydrogen, $R^2$ and $R^3$ each is hydrogen or lower alkyl, $R^4$ is $NR^6R^7$ wherein $R^6$ and $R^7$ each is lower alkyl and $R^5$ is hydrogen, or $R^4$ is hydrogen and $R^5$ is $NR^6R^7$ wherein $R^6$ and $R^7$ each is lower alkyl, and

(h) when a therapeutically acceptable acid addition salt of a compound of formula I is required, reacting the compound of formula I with a therapeutically acceptable acid.

10. A compound of the formula

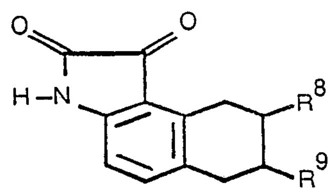

(X)

in which $R^8$ is $NR^{10}R^{11}$ wherein $R^{10}$ and $R^{11}$ each is benzyl or lower alkyl of 1 to 5 carbon atoms and $R^9$ is hydrogen, or $R^8$ is hydrogen and $R^9$ is $NR^{10}R^{11}$ wherein $R^{10}$ and $R^{11}$ each is benzyl or lower alkyl of 1 to 5 carbon atoms.

11. A process for preparing a compound of formula X as defined in Claim 10 which comprises cyclising, according to the procedure of the Sandmeyer Isonitroso acetanilide Isatin reaction, a compound of formula IX:

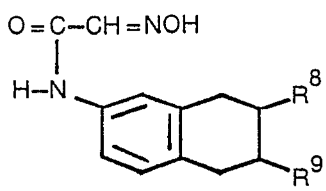

(IX)

wherein $R^8$ and $R^9$ are as defined in Claim 10.

**Claims for the Contracting State: AT**

1. A process for preparing a compound of formula I

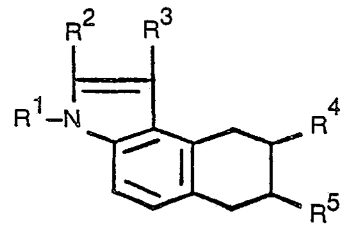

(I)

in which $R^1$, $R^2$ and $R^3$ each is hydrogen or lower alkyl having 1 to 5 carbon atoms, $R^4$ is $NR^6R^7$ wherein $R^6$ and $R^7$ each is hydrogen or lower alkyl having 1 to 5 carbon atoms and $R^5$ is hydrogen, or $R^4$ is hydrogen and $R^5$ is $NR^6R^7$ wherein $R^6$ and $R^7$ each is hydrogen or lower alkyl having 1 to 5 carbon atoms, or a therapeutically acceptable acid addition salt thereof, which comprises one of the following:

(a) when a compound of formula I in which $R^1$, $R^2$ and $R^3$ are hydrogen, $R^4$ is $NR^6R^7$ wherein $R^6$ and $R^7$ each is lower alkyl having 1 to 5 carbon atoms and $R^5$ is hydrogen, or $R^4$ is hydrogen and $R^5$ is $NR^6R^7$ wherein $R^6$ and $R^7$ each is lower alkyl having 1 to 5 carbon atoms is required, reducing a corresponding compound of formula X

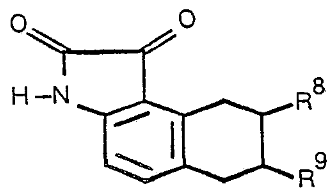

(X)

in which $R^8$ is $NR^{10}R^{11}$ wherein $R^{10}$ and $R^{11}$ each is lower alkyl and $R^9$ is hydrogen, or $R^8$ is hydrogen and $R^9$ is $NR^{10}R^{11}$ wherein $R^{10}$ and $R^{11}$ each is lower alkyl with a complex metal hydride;

(b) when a compound of formula I in which $R^1$ is hydrogen or lower alkyl, $R^2$ and $R^3$ are hydrogen, $R^4$ is $NR^6R^7$ wherein $R^6$ is hydrogen and $R^7$ is hydrogen or lower alkyl and $R^5$ is hydrogen, or $R^4$ is hydrogen and $R^5$ is $NR^6R^7$ wherein $R^6$ is hydrogen and $R^7$ is hydrogen or lower alkyl is required, hydrogenating a compound of formula XI

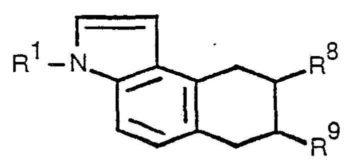

(XI)

in which $R^1$ is hydrogen or lower alkyl, $R^8$ is $NR^{10}R^{11}$ wherein $R^{10}$ is benzyl and $R^{11}$ is benzyl or lower alkyl and $R^9$ is hydrogen, or $R^8$ is hydrogen and $R^9$ is $NR^{10}R^{11}$ wherein $R^{10}$ is benzyl and $R^{11}$ is benzyl or lower alkyl;

(c) when a compound of formula I in which $R^1$ is hydrogen, $R^2$ is lower alkyl, $R^3$ is hydrogen or lower alkyl, $R^4$ is $NR^6R^7$ wherein $R^6$ and $R^7$ each is lower alkyl and $R^5$ is hydrogen, or $R^4$ is hydrogen and $R^5$ is $NR^6R^7$ wherein $R^6$ and $R^7$ each is lower alkyl is required, condensing a compound of formula XII

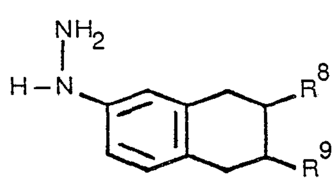

(XII)

in which $R^8$ is $NR^{10}R^{11}$ wherein $R^{10}$ and $R^{11}$ each is lower alkyl and $R^9$ is hydrogen, or $R^8$ is hydrogen and $R^9$ is $NR^{10}R^{11}$ wherein $R^{10}$ and $R^{11}$ each is lower alkyl with a ketone of the formula

$$R^2-\underset{\underset{O}{\|}}{C}-CH_2-R^3$$

in which $R^2$ is lower alkyl and $R^3$ is hydrogen or lower alkyl according to the Fischer indole method;

(d) when a compound of formula I in which $R^1$ and $R^3$ each is hydrogen or lower alkyl, $R^2$ is lower alkyl, $R^4$ is $NR^6R^7$ wherein $R^6$ is hydrogen and $R^7$ is hydrogen or lower alkyl and $R^5$ is hydrogen, or $R^4$ is hydrogen and $R^5$ is $NR^6R^7$ wherein $R^6$ is hydrogen and $R^7$ is hydrogen or lower alkyl is required, hydrogenating a corresponding compound of formula XIV

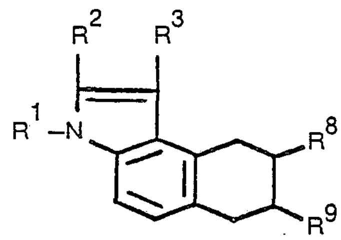

(XIV)

in which $R^1$ and $R^3$ each is hydrogen or lower alkyl, $R^2$ is lower alkyl, $R^8$ is $NR^{10}R^{11}$ wherein $R^{10}$ is benzyl and $R^{11}$ is benzyl or lower alkyl and $R^9$ is hydrogen, or $R^8$ is hydrogen and $R^9$ is $NR^{10}R^{11}$ wherein $R^{10}$ is benzyl and $R^{11}$ is benzyl or lower alkyl;

(e) when a compound of formula I in which $R^1$ and $R^2$ are hydrogen, $R^3$ is lower alkyl, $R^4$ is $NR^6R^7$ wherein $R^6$ and $R^7$ each is lower alkyl and $R^5$ is hydrogen, or $R^4$ is hydrogen and $R^5$ is $NR^6R^7$ wherein $R^6$ and $R^7$ each is lower alkyl is required, decarboxylating a corresponding compound of formula XVI

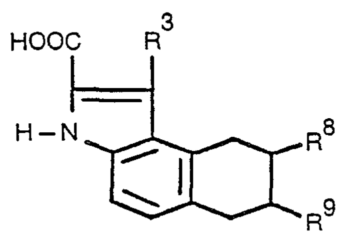

(XVI)

in which $R^3$ is lower alkyl, $R^8$ is $NR^{10}R^{11}$ wherein $R^{10}$ and $R^{11}$ each is lower alkyl and $R^9$ is hydrogen, or $R^8$ is hydrogen and $R^9$ is $NR^{10}R^{11}$ wherein $R^{10}$ and $R^{11}$ each is lower alkyl;

(f) when a compound of formula I in which $R^1$ is hydrogen or lower alkyl, $R^2$ is hydrogen, $R^3$ is lower alkyl, $R^4$ is $NR^6R^7$ wherein $R^6$ is hydrogen and $R^7$ is hydrogen or lower alkyl and $R^5$ is hydrogen, or $R^4$ is hydrogen and $R^5$ is $NR^6R^7$ wherein $R^6$ is hydrogen and $R^7$ is hydrogen or lower alkyl is required, hydrogenating a corresponding compound of formula XVII

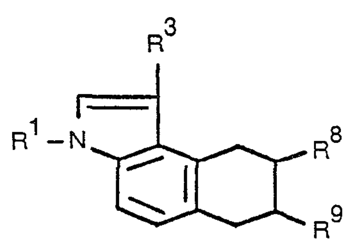

(XVII)

in which $R^1$ is hydrogen or lower alkyl, $R^3$ is lower alkyl, $R^8$ is $NR^{10}R^{11}$ wherein $R^{10}$ is benzyl and $R^{11}$ is benzyl or lower alkyl and $R^9$ is hydrogen, or $R^8$ is hydrogen and $R^9$ is $NR^{10}R^{11}$ wherein $R^{10}$ is benzyl and $R^{11}$ is benzyl or lower alkyl;

(g) when a compound of formula I in which $R^1$ is lower alkyl, $R^2$ and $R^3$ each is hydrogen or lower alkyl, $R^4$ is $NR^6R^7$ wherein $R^6$ and $R^7$ each is lower alkyl and $R^5$ is hydrogen, or $R^4$ is hydrogen and $R^5$ is $NR^6R^7$ wherein $R^6$ and $R^7$ each is lower alkyl, is required, alkylating the corresponding compound of formula I in which $R^1$ is hydrogen, $R^2$ and $R^3$ each is hydrogen or lower alkyl, $R^4$ is $NR^6R^7$ wherein $R^6$ and $R^7$ each is lower alkyl and $R^5$ is hydrogen, or $R^4$ is hydrogen and $R^5$ is $NR^6R^7$ wherein $R^6$ and $R^7$ each is lower alkyl, and

(h) when a therapeutically acceptable acid addition salt of a compound of formula I is required, reacting the coompound of formula I with a therapeutically acceptable acid.

2. A process as claimed in claim 1 for preparing a compound of Formula I wherein $R^1$, $R^2$ and $R^3$

each is hydrogen or lower alkyl having 1 to 3 carbon atoms, $R^4$ is $NR^6R^7$ wherein $R^6$ and $R^7$ each is hydrogen or lower alkyl having 1 to 3 carbon atoms and $R^5$ is hydrogen, or $R^4$ is hydrogen and $R^5$ is $NR^6R^7$ wherein $R^6$ and $R^7$ each is hydrogen or lower alkyl having 1 to 3 carbon atoms, or a therapeutically acceptable acid addition salt thereof.

3. A process as claimed in claim 1 for preparing a compound of Formula I wherein $R^1$, $R^2$ and $R^3$ are hydrogen, $R^4$ is $NR^6R^7$ wherein $R^6$ and $R^7$ each is hydrogen or lower alkyl having 1 to 3 carbon atoms and $R^5$ is hydrogen, or $R^4$ is hydrogen and $R^5$ is $NR^6R^7$ wherein $R^6$ and $R^7$ each is hydrogen or lower alkyl having 1 to 5 carbon atoms, or a therapeutically acceptable acid addition salt thereof.

4. A process for preparing a compound of formula I

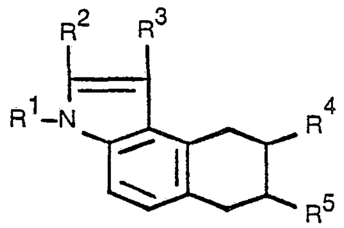

(I)

in which $R^1$ is hydrogen or lower alkyl having 1 to 5 carbon atoms, $R^2$ and $R^3$ are hydrogen, $R^4$ is $NR^6R^7$ wherein $R^6$ and $R^7$ each is lower alkyl having 1 to 5 carbon atoms and $R^5$ is hydrogen, or $R^4$ is hydrogen and $R^5$ is $NR^6R^7$ wherein $R^6$ and $R^7$ each is lower alkyl having 1 to 5 carbon atoms, or a therapeutically acceptable acid addition salt thereof, which comprises selecting a process from the group of:

(a) when a compound of formula I in which $R^1$, $R^2$ and $R^3$ are hydrogen, $R^4$ is $NR^6R^7$ wherein $R^6$ and $R^7$ each is lower alkyl having 1 to 5 carbon atoms and $R^5$ is hydrogen, or $R^4$ is hydrogen and $R^5$ is $NR^6R^7$ wherein $R^6$ and $R^7$ each is lower alkyl having 1 to 5 carbon atoms is required, reducing a corresponding compound of formula X

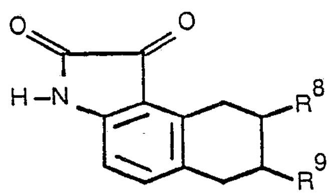

(X)

in which $R^8$ is $NR^{10}R^{11}$ wherein $R^{10}$ and $R^{11}$ each is lower alkyl and $R^9$ is hydrogen, or $R^8$ is hydrogen and $R^9$ is $NR^{10}R^{11}$ wherein $R^{10}$ and $R^{11}$ each is lower alkyl with a complex metal hydride;

(b) when a compound of formula I in which $R^1$ is lower alkyl, $R^2$ and $R^3$ are hydrogen, $R^4$ is $NR^6R^7$ wherein $R^6$ and $R^7$ each is lower alkyl and $R^5$ is hydrogen, or $R^4$ is hydrogen and $R^5$ is $NR^6R^7$ wherein $R^6$ and $R^7$ each is lower alkyl, is required, alkylating the corresponding compound of formula I in which $R^1$, $R^2$ and $R^3$ are hydrogen, $R^4$ is $NR^6R^7$ wherein $R^6$ and $R^7$ each is lower alkyl and $R^5$ is hydrogen, or $R^4$ is hydrogen and $R^5$ is $NR^6R^7$ wherein $R^6$ and $R^7$ each is lower alkyl, and

(c) when a therapeutically acceptable acid addition salt of a compound of formula I is required, reacting the compound of formula I with a therapeutically acceptable acid.

5. A process as claimed in claim 4, for preparing a compound of formula I wherein $R^1$, $R^2$ and $R^3$ are hydrogen, $R^4$ is $NR^6R^7$ wherein $R^6$ and $R^7$ each is lower alkyl having 1 to 3 carbon atoms and $R^5$ is hydrogen, or $R^4$ is hydrogen and $R^5$ is $NR^6R^7$ wherein $R^6$ and $R^7$ each is lower alkyl having 1 to 3 carbon atoms, or a therapeutically acceptable acid addition salt thereof.

6. A process as claimed in claim 4, for preparing a compound of formula I wherein $R^1$ is hydrogen or methyl; $R^2$, $R^3$ and $R^5$ are hydrogen; and $R^4$ is $NR^6R^7$ wherein $R^6$ and $R^7$ are propyl.

7. A process as claimed in claim 4, for preparing a compound of formula I wherein $R^1$, $R^2$, $R^3$ and $R^4$ are hydrogen, and $R^5$ is $NR^6R^7$ wherein $R^6$ and $R^7$ are propyl.

8. A process as claimed in claim 4, for preparing a compound of formula I wherein $R^1$ is hydrogen or methyl; $R^2$, $R^3$ and $R^5$ are hydrogen; and $R^4$ is $NR^6R^7$ wherein $R^6$ and $R^7$ are methyl.

9. The process of claim 1 or 4 wherein the complex metal hydride is lithium aluminium hydride.

10. A process for preparing a pharmaceutical composition characterised in that a compound of formula I as defined in Claim 1 or a therapeutically acceptable salt thereof is mixed with a pharmaceutical carrier and brought into a form suitable for therapeutical administration.

11. A process for preparing a compound of formula

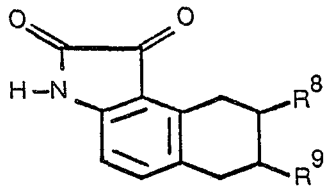

(X)

in which $R^8$ is $NR^{10}R^{11}$ wherein $R^{10}$ and $R^{11}$ each is benzyl or lower alkyl of 1 to 5 carbon atoms and $R^9$ is hydrogen, or $R^8$ is hydrogen and $R^9$ is $NR^{10}R^{11}$ wherein $R^{10}$ and $R^{11}$ each is benzyl or lower alkyl of 1 to 5 carbon atoms which comprises cyclising, according to the procedure of the Sandmeyer Isonitrosoacetanilide Isatin reaction, a compound of formula IX

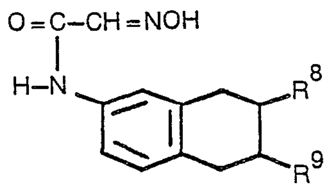

(IX)

wherein $R^8$ and $R^9$ are as defined herein above.

**Patentansprüche fur die Vertragsstaaten: BE CH DE FR GB IT LI LU NL SE**

1. Eine Verbindung der Formel (I)

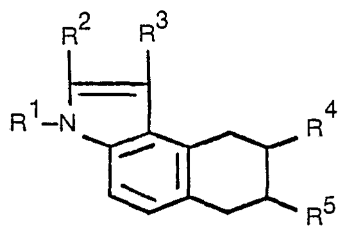

(I)

worin $R^1$, $R^2$ und $R^3$ jeweils Wasserstoff oder nied. Alkyl mit 1 bis 5 C-Atomen sind, $R^4$ die Bedeutung $NR^6R^7$ hat, wobei $R^6$ und $R^7$ jeweils Wasserstoff oder nied.Alkyl mit 1 bis 5 C-Atomen darstellen, und $R^5$ Wasserstoff ist oder $R^4$ Wasserstoff bedeutet und $R^5$ die Bedeutung $NR^6R^7$ hat, wobei $R^6$ und $R^7$ wie hier definiert sind, oder ein therapeutisch annehmbares Säureadditionssalz hievon.

2. Eine Verbindung von Anspruch 1, worin $R^1$, $R^2$ und $R^3$ jeweils Wasserstoff oder nied.Alkyl mit 1 bis 3 C-Atomen bedeuten, $R^4$ die Bedeutung $NR^6R^7$ hat, wobei $R^6$ und $R^7$ jeweils Wasserstoff oder nied.Alkyl mit 1 bis 3 C-Atomen sind, und $R^5$ Wasserstoff ist oder $R^4$ Wasserstoff bedeutet und $R^5$ die Bedeutung $NR^6R^7$ hat, wobei $R^6$ und $R^7$ jeweils Wasserstoff oder nied.Alkyl mit 1 bis 3 C-Atomen sind, oder ein therapeutisch annehmbares Säureadditionssalz hievon.

3. Eine Verbindung wie in Anspruch 1 oder Anspruch 2 beansprucht, worin $R^1$, $R^2$ und $R^3$ Wasserstoff sind.

4. Eine Verbindung, wie in Anspruch 1 beansprucht, worin $R^1$, $R^2$ und $R^3$ Wasserstoff sind, $R^4$ die Bedeutung $NR^6R^7$ hat, wobei $R^6$ und $R^7$ jeweils nied.Alkyl mit 1 bis 3 C-Atomen darstellen, und $R^5$ Wasserstoff ist oder $R^4$ Wasserstoff bedeutet und $R^5$ die Bedeutung $NR^6R^7$ hat, wobei $R^6$ und $R^7$ jeweils nied.Alkyl mit 1 bis 3 C-Atomen darstellen, oder ein therapeutisch annehmbares Säureadditionssalz hievon.

5. Eine Verbindung, wie in Anspruch 1 beansprucht, die 6,7,8,9-Tetrahydro-N,N-dipropyl-3H-benz[e]indol-7-amin; 6,7,8,9, - Tetrahydro - N,N - Dipropyl - 3H - benz[e]indol - 8 - amin; 6,7,8,9 - Tetrahydro - N,N - dimethyl - 3H - benz[e]indol - 8 - amin; oder ein therapeutisch annehmbares Säureadditionssalz hievon ist.

6. Eine Verbindung der Formel (I), die 6,7,8,9-Tetrahydro-N,N,3-trimethyl-3H-benz[e]indol-8-amin oder 6,7,8,9 - Tetrahydro - 3 - methyl - N,N - dipropyl - 3H - benz[e]indol - 8 - amin oder ein therapeutisch annehmbares Säureadditionssalz hievon ist.

7. Eine pharmazeutische Zusammensetzung, die eine Verbindung, wie in einem der Ansprüche 1 bis 6 beansprucht, oder ein therapeutisch annehmbares Säureadditionssalz hievon und einen pharmazeutisch annehmbaren Träger hiefür aufweist.

8. Eine Verbindung, wie in einem der Ansprüche 1 bis 6 beansprucht, oder ein thereapeutisch annehmbares Säureadditionssalz hievon zur Verwendung als pharmazeutisches Mittel.

9. Verfahren zum Herstellen einer Verbindung der Formel (I), wie in Anspruch 1 definiert, oder eines therapeutisch annehmbaren Säureadditionssalzes hievon, welches eines der folgenden umfaßt:

(a) wenn eine Verbindung der Formel (I) gewünscht wird, worin $R^1$, $R^2$ und $R^3$ Wasserstoff sind, $R^4$ $NR^6R^7$ bedeutet, wobei $R^6$ und $R^7$ jeweils nied.Alkyl mit 1 bis 5 C-Atomen sind, und $R^5$ Wasserstoff bedeutet oder $R^4$ Wasserstoff ist und $R^5$ $NR^6R^7$ darstellt, wobei $R^6$ und $R^7$ jeweils nied.Alkyl mit 1 bis 5 C-Atomen sind, Reduzieren einer entsprechenden Verbindung der Formel (X)

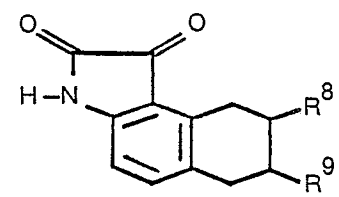

(X)

worin $R^8$ die Bedeutung $NR^{10}R^{11}$ hat, wobei $R^{10}$ und $R^{11}$ jeweils nied.Alkyl sind, und $R^9$ Wasserstoff darstellt oder $R^8$ Wasserstoff ist und $R^9$ die Bedeutung $NR^{10}R^{11}$ hat, wobei $R^{10}$ und $R^{11}$ jeweils nied.Alkyl sind, mit einem komplexen Metallhydrid;

(b) wenn eine Verbindung der Formel (I) gewünscht wird, worin $R^1$ Wasserstoff oder nied.Alkyl ist, $R^2$ und $R^3$ Wasserstoff bedeuten, $R^4$ $NR^6R^7$ darstellt, wobei $R^6$ Wasserstoff und $R^7$ Wasserstoff oder nied.Alkyl sind, udn $R^5$ Wasserstoff bedeutet oder $R^4$ Wasserstoff ist und $R^5$ $NR^6R^7$ darstellt, wobei $R^6$ Wasserstoff und $R^7$ Wasserstoff oder nied.Alkyl sind, Hydrieren einer Verbindung der Formel (XI)

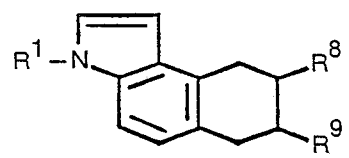

(XI)

worin $R^1$ Wasserstoff oder nied.Alkyl ist, $R^8$ die Bedeutung $NR^{10}R^{11}$ hat, wobei $R^{10}$ Benzyl und $R^{11}$ Benzyl oder nied.Alkyl sind, und $R^9$ Wasserstoff darstellt oder $R^8$ Wasserstoff ist und $R^9$ die Bedeutung $NR^{10}R^{11}$ hat, wobei $R^{10}$ Benzyl und $R^{11}$ Benzyl oder nied.Alkyl sind;

(c) wenn eine Verbindung der Formel (I) gewünscht wird, worin $R^1$ Wasserstoff ist, $R^2$ nied.Alkyl bedeutet, $R^3$ Wasserstoff oder nied.Alkyl darstellt, $R^4$ die Bedeutung $NR^6R^7$ hat, wobei $R^6$ und $R^7$ jeweils nied.Alkyl sind, und $R^5$ Wasserstoff darstellt oder $R^4$ Wasserstoff ist und $R^5$ die Bedeutung $NR^6R^7$ hat, wobei $R^6$ und $R^7$ jeweils nied. Alkyl sind, Kondensieren einer Verbindung der Formel (XII)

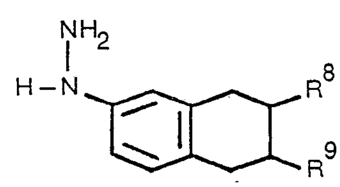

(XII)

worin $R^8$ die Bedeutung $NR^{10}R^{11}$ hat, wobei $R^{10}$ und $R^{11}$ jeweils nied.Alkyl sind, und $R^9$ Wasserstoff bedeutet oder $R^8$ Wasserstoff darstellt und $R^9$ die Bedeutung $NR^{10}R^{11}$ hat, wobei $R^{10}$ und $R^{11}$ jeweils nied.Alkyl sind, mit einem Keton der Formel

$$R^2—\underset{\underset{O}{\|}}{C}—CH_2—R^3,$$

worin $R^2$ nied.Alkyl und $R^3$ Wasserstoff oder nied.Alkyl sind, gemäß der Fischer-Indol-Methode;

(d) wenn eine Verbindung der Formel (I) gewünscht wird, worin $R^1$ und $R^3$ jeweils Wasserstoff oder nied.Alkyl sind, $R^2$ nied.Alkyl bedeutet, $R^4$ die Bedeutung $NR^6R^7$ hat, wobei $R^6$ Wasserstoff und $R^7$ Wasserstoff oder nied.Alkyl sind, und $R^5$ Wasserstoff ist oder $R^4$ Wasserstoff bedeutet und $R^5$ die

Bedeutung $NR^6R^7$ hat, wobei $R^6$ Wasserstoff und $R^7$ Wasserstoff oder nied.Alkyl darstellen, Hydrieren einer entsprechenden Verbindung der Formel (XIV)

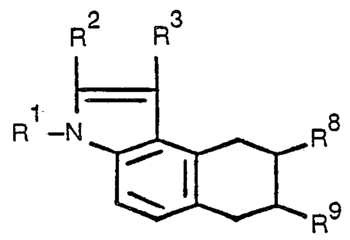

(XIV)

worin $R^1$ und $R^3$ jeweils Wasserstoff oder nied.Alkyl sind, $R^2$ nied.Alkyl bedeutet, $R^8$ $NR^{10}R^{11}$ ist, wobei $R^{10}$ Benzyl und $R^{11}$ Benzyl oder nied.Alkyl darstellen, und $R^9$ Wasserstoff bedeutet oder $R^8$ Wasserstoff ist und $R^9$ die Bedeutung $NR^{10}R^{11}$ hat, wobei $R^{10}$ Benzyl und $R^{11}$ Benzyl oder nied.Alkyl sind;

(e) wenn eine Verbindung der Formel (I) gewünscht wird, worin $R^1$ und $R^2$ Wasserstoff sind, $R^3$ nied.Alkyl bedeutet, $R^4$ die Bedeutung $NR^6R^7$ hat, wobei $R^6$ und $R^7$ jeweils nied.Alkyl sind, und $R^5$ Wasserstoff darstellt oder $R^4$ Wasserstoff ist und $R^5$ die Bedeutung $NR^6R^7$ hat, wobei $R^6$ und $R^7$ jeweils nied.Alkyl sind, Decarboxylieren einer entsprechenden Verbindung der Formel (XVI)

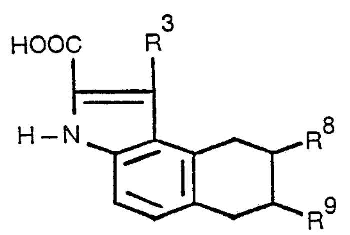

(XVI)

worin $R^3$ nied.Alkyl ist, $R^8$ die Bedeutung $NR^{10}R^{11}$ hat, wobei $R^{10}$ und $R^{11}$ jeweils nied.Alkyl sind, und $R^9$ Wasserstoff darstellt oder $R^8$ Wasserstoff ist und $R^9$ die Bedeutung $NR^{10}R^{11}$ hat, wobei $R^{10}$ und $R^{11}$ jeweils nied.Alkyl bedeuten;

(f) wenn eine Verbindung der Formel (I) gewünscht wird, worin $R^1$ Wasserstoff oder nied.Alkyl ist, $R^2$ Wasserstoff bedeutet, $R^3$ nied.Alkyl darstellt, $R^4$ die Bedeutung $NR^6R^7$ hat, wobei $R^6$ Wasserstoff und $R^7$ Wasserstoff oder nied.Alkyl sind, und $R^5$ Wasserstoff bedeutet oder $R^4$ Wasserstoff ist und $R^5$ die Bedeutung $NR^6R^7$ hat, wobei $R^6$ Wasserstoff und $R^7$ Wasserstoff oder nied.Alkyl sind, Hydrieren einer entsprechenden Verbindung der Formel (XVII)

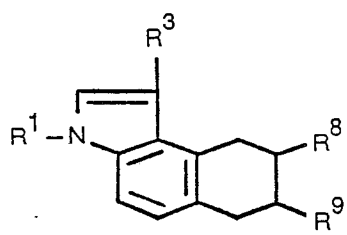

(XVII)

worin $R^1$ Wasserstoff oder nied.Alkyl ist, $R^3$ nied.Alkyl bedeutet, $R^8$ die Bedeutung $NR^{10}R^{11}$ hat, wobei $R^{10}$ Benzyl und $R^{11}$ Benzyl oder nied.Alkyl sind, und $R^9$ Wasserstoff darstellt oder $R^8$ Wasserstoff ist und $R^9$ die Bedeutung $NR^{10}R^{11}$ hat, wobei $R^{10}$ Benzyl und $R^{11}$ Benzyl oder nied.Alkyl sind;

(g) wenn eine Verbindung der Formel (I) gewünscht wird, worin $R^1$ nied.Alkyl ist, $R^2$ und $R^3$ jeweils Wasserstoff oder nied.Alkyl sind, $R^4$ die Bedeutung $NR^6R^7$ hat, wobei $R^6$ und $R^7$ jeweils nied.-Alkyl bedeuten, und $R^5$ Wasserstoff ist oder $R^4$ Wasserstoff ist und $R^5$ die Bedeutung $NR^6R^7$ hat, wobei $R^6$ und $R^7$ jeweils nied.Alkyl sind, Alkylieren der entsprechenden Verbindung der Formel (I), worin $R^1$ Wasserstoff ist, $R^2$ und $R^3$ jeweils Wasserstoff oder nied.-Alkyl bedeuten, $R^4$ die Bedeutung $NR^6R^7$ hat, wobei $R^6$ und $R^7$ jeweils nied.Alkyl sind, und $R^5$ Wasserstoff darstellt oder $R^4$ Wasserstoff ist und $R^5$ die Bedeutung $NR^6R^7$ hat, wobei $R^6$ und $R^7$ jeweils nied.Alkyl sind; und

(h) wenn ein therapeutisch annehmbares Säureadditionssalz einer Verbindung der Formel (I) gewünscht wird, Umsetzen der Verbindung der Formel (I) mit einer therapeutisch annehmbaren Säure.

10. Eine Verbindung der Formel

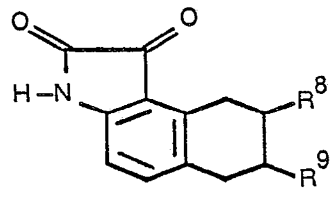

(X)

worin $R^8$ die Bedeutung $NR^{10}R^{11}$ hat, wobei $R^{10}$ und $R^{11}$ jeweils Benzyl oder nied.Alkyl mit 1 bis 5 C-Atomen bedeutet, und $R^9$ Wasserstoff ist oder $R^8$ Wasserstoff darstellt und $R^9$ die Bedeutung $NR^{10}R^{11}$ hat, wobei $R^{10}$ und $R^{11}$ jeweils Benzyl oder nied.Alkyl mit 1 bis 5 C-Atomen sind.

11. Verfahren zum Herstellen einer Verbindung der Formel (X), wie in Anspruch 10 definiert, welches das Cyclisieren entsprechend dem Verfahren der Sandmeyer-Isonitroso-acetanilid-Isatin-Reaktion einer Verbindung der Formel (IX)

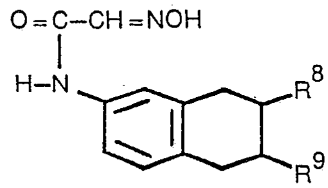

(IX)

worin $R^8$ und $R^9$ wie in Anspruch 10 definiert sind, umfaßt.

**Patentansprüche für den Vertragsstaat: AT**

1. Verfahren zum Herstellen einer Verbindung der Formel (I)

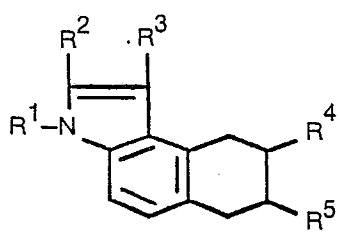

(I)

worin $R^1$, $R^2$ und $R^3$ jeweils Wasserstoff oder nied.Alkyl mit 1 bis 5 C-Atomen sind, $R^4$ die Bedeutung $NR^6R^7$ hat, wobei $R^6$ und $R^7$ jeweils Wasserstoff oder nied.Alkyl mit 1 bis 5 C-Atomen bedeuten, und $R^5$ Wasserstoff darstellt oder $R^4$ Wasserstoff ist und $R^5$ die Bedeutung $NR^6R^7$ hat, wobei $R^6$ und $R^7$ jeweils Wasserstoff oder nied.Alkyl mit 1 bis 5 C-Atomen bedeuten, oder eines therapeutisch annehmbaren Säureadditionssalzes hievon, welches eines der folgenden umfaßt:

(a) wenn eine Verbindung der Formel (I) gewünscht wird, worin $R^1$, $R^2$ und $R^3$ Wasserstoff sind, $R^4$ $NR^6R^7$ bedeutet, wobei $R^6$ und $R^7$ jeweils nied.Alkyl mit 1 bis 5 C-Atomen sind, und $R^5$ Wasserstoff bedeutet oder $R^4$ Wasserstoff ist und $R^5$ $NR^6R^7$ darstellt wobei $R^6$ und $R^7$ jeweils nied.Alkyl mit 1 bis 5 C-Atomen sind, Reduzieren einer entsprechenden Verbindung der Formel (X)

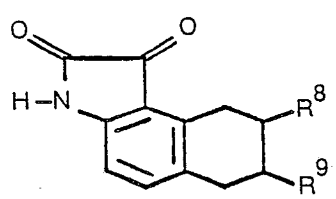

(X)

worin $R^8$ die Bedeutung $NR^6R^7$ hat, wobei $R^{10}$ und $R^{11}$ jeweils nied.Alkyl sind, und $R^9$ Wasserstoff darstellt oder $R^8$ Wasserstoff ist und $R^9$ die Bedeutung $NR^{10}R^{11}$ hat, wobei $R^{10}$ und $R^{11}$ jeweils nied.Alkyl sind, mit einem komplexen Metallhydrid;

(b) wenn eine Verbindung der Formel (I) gewünscht wird, worin $R^1$ Wasserstoff oder nied.Alkyl ist, $R^2$ und $R^3$ Wasserstoff bedeuten, $R^4$ $NR^6R^7$ darstellt, wobei $R^6$ Wasserstoff und $R^7$ Wasserstoff oder nied.Alkyl sind, und $R^5$ Wasserstoff bedeutet oder $R^4$ Wasserstoff ist und $R^5$ $NR^6R^7$ darstellt, wobei $R^6$ Wasserstoff und $R^7$ Wasserstoff oder nied.Alkyl sind, Hydrieren einer Verbindung der Formel (XI)

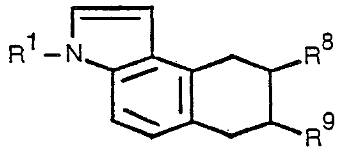

(XI)

worin $R^1$ Wasserstoff oder nied.Alkyl ist, $R^8$ die Bedeutung $NR^{10}R^{11}$ hat, wobei $R^{10}$ Benzyl und $R^{11}$ Benzyl oder nied.Alkyl sind, und $R^9$ Wasserstoff darstellt oder $R^8$ Wasserstoff ist, und $R^9$ die Bedeutung $NR^{10}R^{11}$ hat, wobei $R^{10}$ Benzyl und $R^{11}$ Benzyl oder nied.Alkyl sind:

(c) wenn eine Verbindung der Formel (I) gewünscht wird, worin $R^1$ Wasserstoff ist, $R^2$ nied.Alkyl bedeutet, $R^3$ Wasserstoff oder nied.Alkyl darstellt, $R^4$ die Bedeutung $NR^6R^7$ hat, wobei $R^6$ und $R^7$ jeweils nied.Alkyl sind, und $R^5$ Wasserstoff darstellt oder $R^4$ Wasserstoff ist und $R^5$ die Bedeutung $NR^6R^7$ hat, wobei $R^6$ und $R^7$ jeweils nied.Alkyl sind, Kondensieren einer Verbindung der Formel (XII)

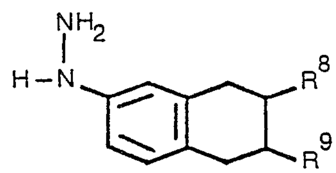

(XII)

worin $R^8$ die Bedeutung $NR^{10}R^{11}$ hat, wobei $R^{10}$ und $R^{11}$ jeweils nied.Alkyl sind, und $R^9$ Wasserstoff bedeutet oder $R^8$ Wasserstoff darstellt und $R^9$ die Bedeutung $NR^{10}R^{11}$ hat, wobei $R^{10}$ und $R^{11}$ jeweils nied.Alkyl sind, mit einem Keton der Formel

$$R^2-\underset{\underset{O}{\|}}{C}-CH_2-R^3,$$

worin $R^2$ nied.Alkyl und $R^3$ Wasserstoff oder nied.Alkyl sind, gemäß der Fischer-Indol-Methode;

(d) wenn eine Verbindung der Formel (I) gewünscht wird, worin $R^1$ und $R^3$ jeweils Wasserstoff oder nied.Alkyl sind, $R^2$ nied.Alkyl bedeutet, $R^4$ die Bedeutung $NR^6R^7$ hat, wobei $R^6$ Wasserstoff und $R^7$ Wasserstoff oder nied.Alkyl sind, und $R^5$ Wasserstoff ist oder $R^4$ Wasserstoff bedeutet und $R^5$ die Bedeutung $NR^6R^7$ hat, wobei $R^6$ Wasserstoff und $R^7$ Wasserstoff oder nied.Alkyl darstellen, Hydrieren einer entsprechenden Verbindung der Formel (XIV)

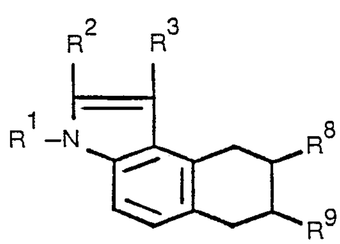

(XIV)

worin $R^1$ und $R^3$ jeweils Wasserstoff oder nied.Alkyl sind, $R^2$ nied.Alkyl bedeutet, $R^8$ $NR^{10}R^{11}$ ist, wobei $R^{10}$ Benzyl und $R^{11}$ Benzyl oder nied.Alkyl darstellen, und $R^9$ Wasserstoff bedeutet oder $R^8$ Wasserstoff ist und $R^9$ die Bedeutung $NR^{10}R^{11}$ hat, wobei $R^{10}$ Benzyl und $R^{11}$ Benzyl oder nied.Alkyl sind;

(e) wenn eine Verbindung der Formel (I) gewünscht wird, worin $R^1$ und $R^2$ Wasserstoff sind, $R^3$ nied.Alkyl bedeutet, $R^4$ die Bedeutung $NR^6R^7$ hat, wobei $R^6$ und $R^7$ jeweils nied.Alkyl sind, und $R^5$ Wasserstoff darstellt oder $R^4$ Wasserstoff ist und $R^5$ die Bedeutung $NR^6R^7$ hat, wobei $R^6$ und $R^7$ jeweils nied.Alkyl sind, Decarboxylieren einer entsprechenden Verbindung der Formel (XVI)

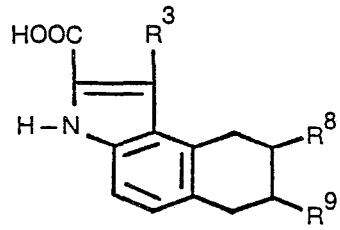

(XVI)

worin $R^3$ nied.Alkyl ist, $R^8$ die Bedeutung $NR^{10}R^{11}$ hat, wobei $R^{10}$ und $R^{11}$ jeweils nied.Alkyl sind, und $R^9$ Wasserstoff darstellt oder $R^8$ Wasserstoff ist und $R^9$ die Bedeutung $NR^{10}R^{11}$ hat, wobei $R^{10}$ und $R^{11}$ jeweils nied.Alkyl bedeuten;

(f) wenn eine Verbindung der Formel (I) gewünscht wird, worin $R^1$ Wasserstoff oder nied.Alkyl ist, $R^2$ Wasserstoff bedeutet, $R^3$ nied.Alkyl darstellt, $R^4$ die Bedeutung $NR^6R^7$ hat, wobei $R^6$ Wasserstoff und $R^7$ Wasserstoff oder nied.Alkyl sind, und $R^5$ Wasserstoff bedeutet oder $R^4$ Wasserstoff ist und $R^5$ die Bedeutung $NR^6R^7$ hat, wobei $R^6$ Wasserstoff und $R^7$ Wasserstoff oder nied.Alkyl sind, Hydrieren einer entsprechenden Verbindung der Formel (XVII)

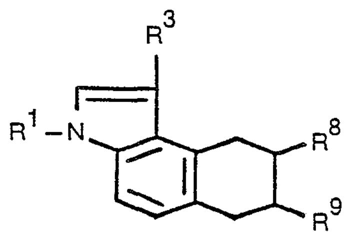

(XVII)

worin $R^1$ Wasserstoff oder nied.Alkyl ist, $R^3$ nied.Alkyl bedeutet, $R^8$ die Bedeutung $NR^{10}R^{11}$ hat, wobei $R^{10}$ Benzyl und $R^{11}$ Benzyl oder nied.Alkyl sind, und $R^9$ Wasserstoff darstellt oder $R^8$ Wasserstoff ist und $R^9$ die Bedeutung $NR^{10}R^{11}$ hat, wobei $R^{10}$ Benzyl und $R^{11}$ Benzyl oder nied.Alkyl sind;

(g) wenn eine Verbindung der Formel (I) gewünscht wird, worin $R^1$ nied.Alkyl ist, $R^2$ und $R^3$ jeweils Wasserstoff oder nied.Alkyl sind, $R^4$ die Bedeutung $NR^6R^7$ hat, wobei $R^6$ und $R^7$ jeweils nied.Alkyl bedeuten, und $R^5$ Wasserstoff ist oder $R^4$ Wasserstoff ist und $R^5$ die Bedeutung $NR^6R^7$ hat, wobei $R^6$ und $R^7$ jeweils nied.Alkyl sind, Alkylieren der entsprechenden Verbindung der Formel (I), worin $R^1$ Wasserstoff ist, $R^2$ und $R^3$ jeweils Wasserstoff oder nied.Alkyl bedeuten, $R^4$ die Bedeutung $NR^6R^7$ hat, wobei $R^6$ und $R^7$ jeweils nied.Alkyl sind, und $R^5$ Wasserstoff darstellt oder $R^4$ Wasserstoff ist und $R^5$ die Bedeutung $NR^6R^7$ hat, wobei $R^6$ und $R^7$ jeweils nied.Alkyl sind; und

(h) wenn ein therapeutisch annehmbares Säureadditionssalz einer Verbindung der Formel (I) gewünscht wird, Umsetzen der Verbindung der Formel (I) mit einer therapeutisch annehmbaren Säure.

2. Verfahren, wie in Anspruch 1 beansprucht, zur Herstellung einer Verbindung der Formel (I), worin $R^1$, $R^2$ und $R^3$ jeweils Wasserstoff oder nied.Alkyl mit 1 bis 3 C-Atomen sind, $R^4$ die Bedeutung $NR^6R^7$ hat, wobei $R^6$ und $R^7$ jeweils Wasserstoff oder nied.Alkyl mit 1 bis 3 C-Atomen sind, und $R^5$ Wasserstoff bedeutet oder $R^4$ Wasserstoff ist und $R^5$ die Bedeutung $NR^6R^7$ hat, wobei $R^6$ und $R^7$ jeweils Wasserstoff oder nied.Alkyl mit 1 bis 3 C-Atomen sind, oder eines therapeutisch annehmbaren Säureadditionssalzes hievon.

3. Verfahren, wie in Anspruch 1 beansprucht, zur Herstellung einer Verbindung der Formel (I), worin $R^1$, $R^2$ und $R^3$ Wasserstoff sind, $R^4$ die Bedeutung $NR^6R^7$ hat, wobei $R^6$ und $R^7$ jeweils Wasserstoff oder nied.Alkyl mit 1 bis 3 C-Atomen bedeuten, und $R^5$ Wasserstoff ist oder $R^4$ Wasserstoff darstellt und $R^5$ die Bedeutung $NR^6R^7$ hat, wobei $R^6$ und $R^7$ jeweils Wasserstoff oder nied.Alkyl mit 1 bis 3 C-Atomen bedeuten, oder eines therapeutisch annehmbaren Säureadditionssalzes hievon.

4. Verfahren zum Herstellen einer Verbindung der Formel (I)

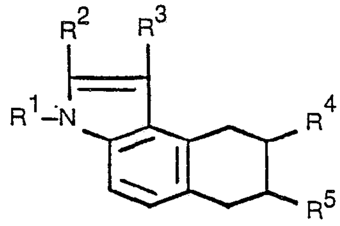

(I)

worin $R^1$ Wasserstoff oder nied.Alkyl mit 1 bis 5 C-Atomen ist, $R^2$ und $R^3$ Wasserstoff bedeuten, $R^4$ die Bedeutung $NR^6R^7$ hat, wobei $R^6$ und $R^7$ jeweils nied.Alkyl mit 1 bis 5 C-Atomen sind, und $R^5$ Wasserstoff darstellt oder $R^4$ Wasserstoff ist und $R^5$ die Bedeutung $NR^6R^7$ hat, wobei $R^6$ und $R^7$ jeweils nied.Alkyl mit 1 bis 5 C-Atomen sind, oder eines therapeutisch annehmbaren Säureadditionssalzes hievon, welches das Auswählen eines Verfahrens aus der Gruppe:

(a) wenn eine Verbindung der Formel (I) gewünscht wird, worin $R^1$, $R^2$ und $R^3$ Wasserstoff sind, $R^4$ die Bedeutung $NR^6R^7$ hat, wobei $R^6$ und $R^7$ jeweils nied.Alkyl mit 1 bis 5 C-Atomen bedeuten, und $R^5$ Wasserstoff ist oder $R^4$ Wasserstoff darstellt und $R^5$ die Bedeutung $NR^6R^7$ hat, wobei $R^6$ und $R^7$ jeweils nied.Alkyl mit 1 bis 5 C-Atomen bedeuten, Reduzieren einer entsprechenden Verbindung der Formel (X)

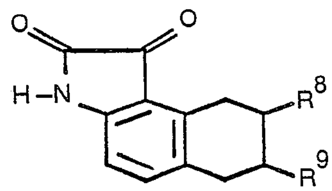
(X)

worin $R^8$ die Bedeutung $NR^{10}R^{11}$ hat, wobei $R^{10}$ und $R^{11}$ jeweils nied.Alkyl sind, und $R^9$ Wasserstoff ist oder $R^8$ Wasserstoff darstellt und $R^9$ Bedeutung $NR^{10}R^{11}$ hat, wobei $R^{10}$ und $R^{11}$ jeweils nied.Alkyl sind, mit einem komplexen Metallhydrid;

(b) wenn eine Verbindung der Formel (I) gewünscht wird, worin $R^1$ nied.Alkyl ist, $R^2$ und $R^3$ Wasserstoff bedeuten, $R^4$ die Bedeutung $NR^6R^7$ hat, wobei $R^6$ und $R^7$ jeweils nied.Alkyl sind, und $R^5$ Wasserstoff ist oder $R^4$ Wasserstoff bedeutet und $R^5$ die Bedeutung $NR^6R^7$ hat, wobei $R^6$ und $R^7$ jeweils nied.Alkyl sind, Alkylieren der entsprechenden Verbindung der Formel (I), worin $R^1$, $R^2$ und $R^3$ Wasserstoff sind, $R^4$ die Bedeutung $NR^6R^7$ hat, wobei $R^6$ und $R^7$ jeweils nied.Alkyl sind, und $R^5$ Wasserstoff ist oder $R^4$ Wasserstoff darstellt und $R^5$ die Bedeutung $NR^6R^7$ hat, wobei $R^6$ und $R^7$ jeweils nied.Alkyl sind, und

(c) wenn eine therapeutisch annehmbares Säureadditionssalz einer Verbindung der Formel (I) gewünscht wird, Umsetzen der Verbindung der Formel (I) mit einer therapeutisch annehmbaren Säure umfaßt.

5. Verfahren, wie in Anspruch 4 beansprucht, zur Herstellung einer Verbindung der Formel (I), worin $R^1$, $R^2$ und $R^3$ jeweils Wasserstoff sind, $R^4$ die Bedeutung $NR^6R^7$ hat, wobei $R^6$ und $R^7$ jeweils nied.Alkyl mit 1 bis 3 C-Atomen sind, und $R^5$ Wasserstoff ist oder $R^4$ Wasserstoff darstellt und $R^5$ die Bedeutung $NR^6R^7$ hat, wobei $R^6$ und $R^7$ jeweils nied.Alkyl mit 1 bis 3 C-Atomen sind, oder eines therapeutisch annehmbaren Säureadditionssalzes hievon.

6. Verfahren, wie in Anspruch 4 beansprucht, zur Herstellung einer Verbindung der Formel (I), worin $R^1$ Wasserstoff oder Methyl ist, $R^2$, $R^3$ und $R^5$ Wasserstoff darstellen und $R^4$ die Bedeutung $NR^6R^7$ hat, wobei $R^6$ und $R^7$ Propyl sind.

7. Verfahren, wie in Anspruch 4 beansprucht, zur Herstellung einer Verbindung der Formel (I), worin $R^1$, $R^2$, $R^3$ und $R^4$ Wasserstoff bedeuten und $R^5$ die Bedeutung $NR^6R^7$ hat, wobei $R^6$ und $R^7$ Propyl sind.

8. Verfahren, wie in Anspruch 4 beansprucht, zur Herstellung einer Verbindung der Formel (I), worin $R^1$ Wasserstoff oder Methyl ist, $R^2$, $R^3$ und $R^5$ Wasserstoff bedeuten, und $R^4$ die Bedeutung $NR^6R^7$ hat, wobei $R^6$ und $R^7$ Methyl darstellen.

9. Verfahren gemäß Anspruch 1 oder 4, worin das komplexe Metallhydrid Lithiumaluminiumhydrid ist.

10. Verfahren zum Herstellen einer pharmazeutischen Zusammensetzung, dadurch gekennzeichnet, daß eine Verbindung der Formel (I), wie in Anspruch 1 definiert, oder ein therapeutisch annehmbares Salz hievon mit einem pharmazeutischen Träger gemischt und in eine für therapeutische Verabreichung geeignete Form gebracht wird.

11. Verfahren zum Herstellen einer Verbindung der Formel

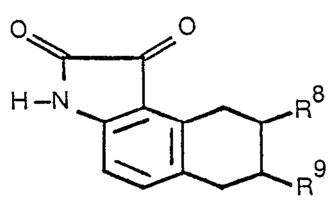
(X)

worin $R^8$ die Bedeutung $NR^{10}R^{11}$ hat, wobei $R^{10}$ und $R^{11}$ jeweils Benzyl oder nied. Alkyl mit 1 bis 5 C-Atomen bedeuten, und $R^9$ Wasserstoff ist oder $R^8$ Wasserstoff darstellt und $R^9$ die Bedeutung $NR^{10}R^{11}$

hat, wobei $R^{10}$ und $R^{11}$ jeweils Benzyl oder nied.Alkyl mit 1 bis 5 C-Atomen sind, welches das Cyclisieren entsprechend dem Verfahren der Sandmeyer-Isonitrosoacetanilid-Isatin-Reaktion einer Verbindung der Formel (IX)

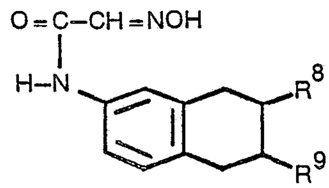

(IX)

worin $R^8$ und $R^9$ wie hier oben definiert sind, umfaßt.

**Revendications pour les etats contractants: BE CH DE FR GB IT LI LU NL SE**

1. Composé de formula I

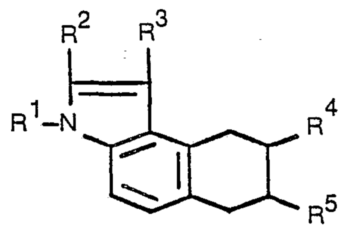

(I)

dans laquelle $R^1$, $R^2$ et $R^3$ représentent chacun l'hydrogène ou un groupe alkyle inférieur ayant 1 à 5 atomes de carbone, $R^4$ est un groupe $NR^6R^7$ dans lequel $R^6$ et $R^7$ représentent chacun l'hydrogène ou un groupe alkyle inférieur ayant 1 à 5 atomes de carbone et $R^5$ est l'hydrogène, ou bien $R^4$ est l'hydrogène et $R^5$ est un groupe $NR^6R^7$ dans lequel $R^6$ et $R^7$ ont les définitions données ci-dessus, ou un sel d'addition d'acide thérapeutiquement acceptable de ce composé.

2. Composé suivant la revendication 1, dans lequel $R^1$, $R^2$ et $R^3$ représentent chacun l'hydrogène ou un groupe alkyle inférieur ayant 1 à 3 atomes de carbone, $R^4$ est un groupe $NR^6R^7$ dans lequel $R^6$ et $R^7$ représentent chacun l'hydrogène ou un groupe alkyle inférieur ayant 1 à 3 atomes de carbone et $R^5$ est l'hydrogène, ou bien $R^4$ est l'hydrogène et $R^5$ est un groupe $NR^6R^7$ dans lequel $R^6$ et $R^7$ représentent chacun l'hydrogène ou un groupe alkyle inférieur ayant 1 à 3 atomes de carbone, ou un sel d'addition d'acide thérapeutiquement acceptable de ce composé.

3. Composé suivant la revendication 1 ou la revendication 2, dans lequel $R^1$, $R^2$ et $R^3$ représentent l'hydrogène.

4. Composé suivant la revendication 1, dans lequel $R^1$, $R^2$ et $R^3$ représentent l'hydrogène, $R^4$ est un groupe $NR^6R^7$ dans lequel $R^6$ et $R^7$ sont chacun un groupe alkyle inférieur ayant 1 à 3 atomes de carbone et $R^5$ est l'hydrogène, ou bien $R^4$ est l'hydrogène et $R^5$ est un groupe $NR^6R^7$ dans lequel $R^6$ et $R^7$ sont chacun un groupe alkyle inférieur ayant 1 à 3 atomes de carbone, ou un sel d'addition d'acide thérapeutiquement acceptable de ce composé.

5. Composé suivant la revendication 1, qui est la 6,7,8,9 - tétrahydro - N,N - dipropyl - 3H - benzo[e]indole - 7 - amine; la 6,7,8,9 - tétrahydro - N,N - dipropyl - 3H - benzo[e]indole - 8 - amine; la 6,7,8,9 - tétrahydro - N,N - diméthyl - 3H - benzo[e]indole - 8 - amine; ou un sel d'addition d'acide thérapeutiquement acceptable de ce composé.

6. Composé de formule I, qui est la 6,7,8,9 - tétrahydro - N,N,3 - triméthyl - 3H - benzo[e]-indole - 8 - amine ou la 6,7,8,9 - tétrahydro - 3 - méthyl - N,N - dipropyl - 3H - benzo[e]-indole - 8 - amine, ou un sel d'addition d'acide thérapeutiquement acceptable de ce composé.

7. Composition pharmaceutiquement, comprenant un composé suivant l'une quelconque des revendications 1 à 6 ou un sel d'addition d'acide thérapeutiquement acceptable de ce composé, et un support pharmaceutiquement acceptable pour ce composé.

8. Composé suivant l'une quelconque des revendications 1 à 6 ou sel d'addition d'acide thérapeutiquement acceptable de ce composé, destiné à être utilisé comme substance pharmaceutique.

9. Procédé de préparation d'un composé de formule I suivant la revendication 1 ou d'un sel d'addition d'acide thérapeutiquement acceptable de ce composé, qui comprend l'une des opérations suivantes:

(a) lorsqu'un composé de formule I dans laquelle $R^1$, $R^2$ et $R^3$ représentent l'hydrogène, $R^4$ est un groupe $NR^6R^7$ dans lequel $R^6$ et $R^7$ sont chacun un radical alkyle inférieur ayant 1 à 5 atomes de carbone et $R^5$ est l'hydrogène, ou bien $R^4$ est l'hydrogène et $R^5$ est un groupe $NR^6R^7$ dans lequel $R^6$ et $R^7$

sont chacun un groupe alkyle inférieur ayant 1 à 5 atomes de carbone, est désiré, réduction d'un composé correspondant de formule X

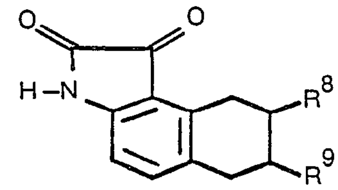

(X)

dans laquelle $R^8$ est un groupe $NR^{10}R^{11}$ où $R^{10}$ et $R^{11}$ représentent chacun un radical alkyle inférieur et $R^9$ est l'hydrogène, ou bien $R^8$ est l'hydrogène et $R^9$ est un groupe $NR^{10}R^{11}$ dans lequel $R^{10}$ et $R^{11}$ sont chacun un groupe alkyle inférieur, avec un hydrure métallique complexe;

(b) lorsqu'un composé de formule I dans laquelle $R^1$ est l'hydrogène ou un radical alkyle inférieur, $R^2$ et $R^3$ représentent l'hydrogène, $R^4$ est un groupe $NR^6R^7$ dans lequel $R^6$ est l'hydrogène, et $R^7$ est l'hydrogène ou un radical alkyle inférieur et $R^5$ est l'hydrogène, ou bien $R^4$ est l'hydrogène et $R^5$ est un groupe $NR^6R^7$ dans lequel $R^6$ est l'hydrogène et $R^7$ est l'hydrogène ou un radical alkyle inférieur, est désiré, hydrogénation d'un composé de formule XI

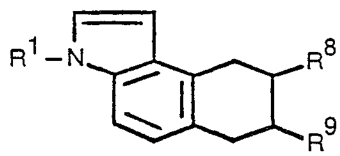

(XI)

dans laquelle $R^1$ est l'hydrogène ou un radical alkyle inférieur, $R^8$ est un groupe $NR^{10}R^{11}$ dans lequel $R^{10}$ est un radical benzyle et $R^{11}$ est un radical benzyle ou alkyle inférieur et $R^9$ est l'hydrogène, ou bien $R^8$ est l'hydrogène et $R^9$ est un groupe $NR^{10}R^{11}$ dans lequel $R^{10}$ est le radical benzyle et $R^{11}$ est un radical benzyle ou alkyle inférieur;

(c) lorsqu'un composé de formule I dans laquelle $R^1$ est l'hydrogène, $R^2$ est un radical alkyle inférieur, $R^3$ est l'hydrogène ou un radical alkyle inférieur, $R^4$ est un groupe $NR^6R^7$ dans lequel $R^6$ et $R^7$ représentent chacun un alkyle inférieur et $R^5$ est l'hydrogène, ou bien $R^4$ est l'hydrogène et $R^5$ est un groupe $NR^6R^7$ dans lequel $R^6$ et $R^7$ représentent chacun un radical alkyle inférieur, est désiré, condensation d'un composé de formule XII

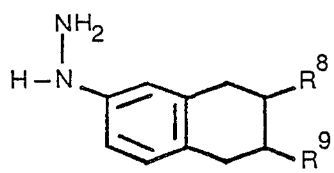

(XII)

dans laquelle $R^8$ est un groupe $NR^{10}R^{11}$ à $R^{10}$ et $R^{11}$ représentent chacun un radical alkyle inférieur et $R^9$ est l'hydrogène, ou bien $R^8$ est l'hydrogène et $R^9$ est un groupe $NR^{10}R^{11}$ dans lequel $R^{10}$ et $R^{11}$ représentent chacun un radical alkyle inférieur, avec une cétone de formule

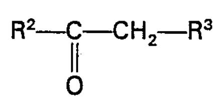

dans laquelle $R^2$ est un radical alkyle inférieur et $R^3$ est l'hydrogène ou un radical alkyle inférieur conformément au procédé à l'indole de Fischer;

(d) lorsqu'un composé de formule I dans laquelle $R^1$ et $R^3$ représentent chacun l'hydrogène ou un radical alkyle inférieur, $R^2$ est un radical alkyle inférieur, $R^4$ est un groupe $NR^6R^7$ dans lequel $R^6$ est l'hydrogène et $R^7$ est l'hydrogène ou un radical alkyle inférieur et $R^5$ est l'hydrogène, ou bien $R^4$ est l'hydrogène et $R^5$ est un groupe $NR^6R^7$ dans lequel $R^6$ est l'hydrogène et $R^7$ est l'hydrogène ou un radical alkyle inférieur, est désiré, hydrogénation d'un composé correspondant de formule XIV

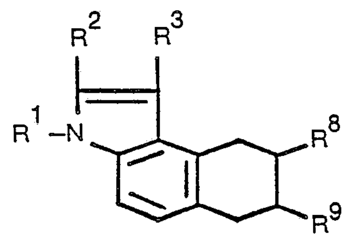

(XIV)

dans laquelle $R^1$ et $R^3$ représentent chacun l'hydrogène ou un radical alkyle inférieur, $R^2$ est un radical alkyle inférieur, $R^8$ est un groupe $NR^{10}R^{11}$ dans lequel $R^{10}$ est un radical benzyle et $R^{11}$ est un radical benzyle ou alkyle inférieur et $R^9$ est l'hydrogène, ou bien $R^8$ est l'hydrogène et $R^9$ est un groupe $NR^{10}R^{11}$ dans lequel $R^{10}$ est un radical benzyle et $R^{11}$ est un radical benzyle ou alkyle inférieur;

(e) lorsqu'un composé de formule I dans laquelle $R^1$ et $R^2$ représentent l'hydrogène, $R^3$ est un radical alkyle inférieur, $R^4$ est un groupe $NR^6R^7$ dans lequel $R^6$ et $R^7$ représentent chacun un radical alkyle inférieur et $R^5$ est l'hydrogène, ou bien $R^4$ est l'hydrogène et $R^5$ est un groupe $NR^6R^7$ dans lequel $R^6$ et $R^7$ représentent chacun un radical alkyle inférieur, est désiré, décarboxylation d'un composé correspondant de formule XVI

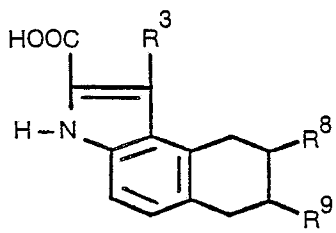

(XVI)

dans laquelle $R^3$ est un radical alkyle inférieur, $R^8$ est un groupe $NR^{10}R^{11}$ dans lequel $R^{10}$ et $R^{11}$ représentent chacun un radical alkyle inférieur et $R^9$ est l'hydrogène, ou bien $R^8$ est l'hydrogène et $R^9$ est un groupe $NR^{10}R^{11}$ dans lequel $R^{10}$ et $R^{11}$ sont chacun un radical alkyle inférieur;

(f) lorsqu'un composé de formule I dans laquelle $R^1$ est l'hydrogène ou un radical alkyle inférieur, $R^2$ est l'hydrogène, $R^3$ est un radical alkyle inférieur, $R^4$ est un groupe $NR^6R^7$ dans lequel $R^6$ est l'hydrogène et $R^7$ est l'hydrogène ou un radical alkyle inférieur et $R^5$ est l'hydrogène, ou bien $R^4$ est l'hydrogène et $R^5$ est un groupe $NR^6R^7$ dans lequel $R^6$ est l'hydrogène et $R^7$ est l'hydrogene ou un radical alkyle inférieur, est désiré, hydrogénation d'un composé correspondant de formule XVII

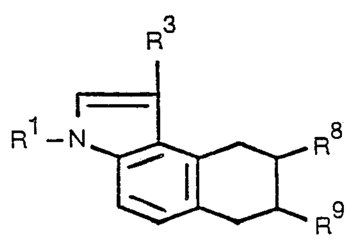

(XVII)

dans laquelle $R^1$ est l'hydrogène ou un radical alkyle inférieur, $R^3$ est un radical alkyle inférieur, $R^8$ est un groupe $NR^{10}R^{11}$ dans lequel $R^{10}$ est un radical benzyle et $R^{11}$ est un radical benzyle ou alkyle inférieur et $R^9$ est l'hydrogène, ou bien $R^8$ est l'hydrogène iet $R^9$ est un groupe $NR^{10}R^{11}$ dans lequel $R^{10}$ est un radical benzyle et $R^{11}$ est un radical benzyle ou alkyle inférieur;

(g) lorsqu'un composé de formule I dans laquelle $R^1$ est un radical alkyle inférieur, $R^2$ et $R^3$ représentent chacun l'hydrogène ou un radical alkyle inférieur, $R^4$ est un groupe $NR^6R^7$ dans lequel $R^6$ et $R^7$ représentent chacun un radical alkyle inférieur et $R^5$ est l'hydrogène, ou bien $R^4$ est l'hydrogène, et $R^5$ est un groupe $NR^6R^7$ dans lequel $R^6$ et $R^7$ représentent chacun un radical alkyle inférieur, est désiré, alkylation du composé correspondant de formule I dans laquelle $R^1$ est l'hydrogène, $R^2$ et $R^3$ sont chacun l'hydrogène ou un radical alkyle inférieur, $R^4$ est un groupe $NR^6R^7$ dans lequel $R^6$ et $R^7$ représentent chacun un radical alkyle inférieur et $R^5$ est l'hydrogène, ou bien $R^4$ est l'hydrogène et $R^5$ est un groupe $NR^6R^7$ dans lequel $R^6$ et $R^7$ sont chacun un groupe alkyle inférieur, et

(h) lorsqu'un sel d'addition d'acide thérapeutiquement acceptable d'un composé de formule I est désiré, réaction du composé de formule I avec un acide thérapeutiquement acceptable.

10. Composé de formule

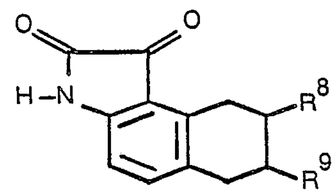

(X)

dans laquelle $R^8$ est un groupe $NR^{10}R^{11}$ où $R^{10}$ et $R^{11}$ représentent chacun un radical benzyle ou alkyle inférieur ayant 1 à 5 atomes de carbone et $R^9$ est l'hydrogène, ou bien $R^8$ est l'hydrogène et $R^9$ est un groupe $NR^{10}R^{11}$ dans lequel $R^{10}$ et $R^{11}$ représentent chacun un radical benzyle ou alkyle inférieur ayant 1 à 5 atomes de carbone.

11. Procédé de préparation d'un composé de formule X suivant la revendication 10, qui comprend la cyclisation, conformément à la réaction de Sandmeyer pour la formation d'isatine avec l'isonitroso-acétanilide, d'un composé de formule IX:

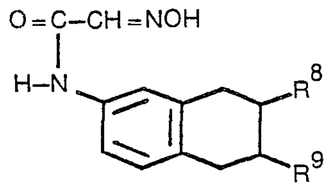

(IX)

dans laquelle $R^8$ et $R^9$ ont les définitions données dans la revendication 10.

**Revendications pour l'etat contractant: AT**

1. Procédé de préparation d'un composé de formule I

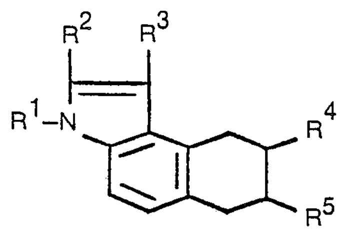

(I)

dans laquelle $R^1$, $R^2$ et $R^3$ sont chacun un atome d'hydrogène ou un radical alkyle inférieur ayant 1 à 5 atomes de carbone, $R^4$ est un groupe $NR^6R^7$ dans lequel $R^6$ et $R^7$ représentent chacun l'hydrogène ou un radical alkyle inférieur ayant 1 à 5 atomes de carbone et $R^5$ est l'hydrogène, ou bien $R^4$ est l'hydrogène et $R^5$ est un groupe $NR^6R^7$ dans lequel $R^6$ et $R^7$ sont chacun un atome d'hydrogène ou un radical alkyle inférieur ayant 1 à 5 atomes de carbone, ou d'un sel d'addition d'acide thérapeutiquement acceptable de ce composé, qui comprend l'une des opérations suivantes:

(a) lorsqu'un composé de formule I dans laquelle $R^1$, $R^2$ et $R^3$ représentent l'hydrogène, $R^4$ est un groupe $NR^6R^7$ dans lequel $R^6$ et $R^7$ sont chacun un radical alkyle inférieur ayant 1 à 5 atomes de carbone et $R^5$ est l'hydrogène, ou bien $R^4$ est l'hydrogène et $R^5$ est un groupe $NR^6R^7$ dans lequel $R^6$ et $R^7$ sont chacun un groupe alkyle inférieur ayant 1 à 5 atomes de carbone, est désiré, réduction d'un composé correspondant de formule X

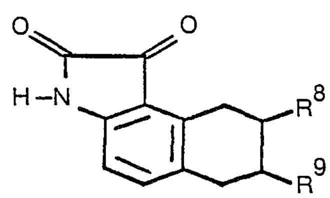

(X)

dans laquelle $R^8$ est un groupe $NR^{10}R^{11}$ où $R^{10}$ et $R^{11}$ représentent chacun un radical alkyle inférieur et $R^9$ est l'hydrogène, ou bien $R^8$ est l'hydrogène et $R^9$ est un groupe $NR^{10}R^{11}$ dans lequel $R^{10}$ et $R^{11}$ sont chacun un groupe alkyle inférieur, avec un hydrure métallique complexe;

(b) lorsqu'un composé de formule I dans laquelle $R^1$ est l'hydrogène ou un radical alkyle inférieur, $R^2$ et $R^3$ représentent l'hydrogène, $R^4$ est un groupe $NR^6R^7$ dans lequel $R^6$ est l'hydrogène et $R^7$ est l'hydrogène ou un radical alkyle inférieur et $R^5$ est l'hydrogène, ou bien $R^4$ est l'hydrogène et $R^5$ est un groupe $NR^6R^7$ dans lequel $R^6$ est l'hydrogène et $R^7$ est l'hydrogène ou un radical alkyle inférieur, est dédiré, hydrogénation d'un composé de formule XI

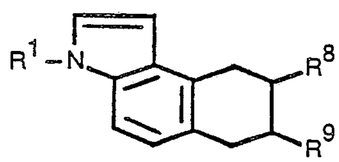

(XI)

dans laquelle $R^1$ est l'hydrogène ou un radical alkyle inférieur, $R^8$ est un groupe $NR^{10}R^{11}$ dans lequel $R^{10}$ est un radical benzyle et $R^{11}$ est un radical benzyle ou alkyle inférieur et $R^9$ est l'hydrogène, ou bien $R^8$ est l'hydrogène et $R^9$ est un groupe $NR^{10}R^{11}$ dans lequel $R^{10}$ est le radical benzyle et $R^{11}$ est un radical benzyle ou alkyle inférieur;

(c) lorsqu'un composé de formule I dans laquelle $R^1$ est l'hydrogène, $R^2$ est un radical alkyle inférieur, $R^3$ est l'hydrogène ou un radical alkyle inférieur, $R^4$ est un groupe $NR^6R^7$ dans lequel $R^6$ et $R^7$ représentent chacun un radical alkyle inférieur et $R^5$ est l'hydrogène, ou bien $R^4$ est l'hydrogène et $R^5$ est un groupe $NR^6R^7$ dans lequel $R^6$ et $R^7$ représentent chacun un radical alkyle inférieur, est désiré, condensation d'un composé de formule XII

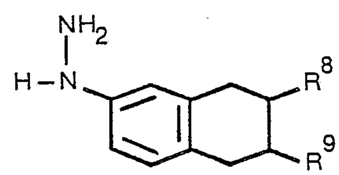

(XII)

dans laquelle $R^8$ est un groupe $NR^{10}R^{11}$ où $R^{10}$ et $R^{11}$ représentent chacun un radical alkyle inférieur et $R^9$ est l'hydrogène, ou bien $R^8$ est l'hydrogène et $R^9$ est un groupe $NR^{10}R^{11}$ dans lequel $R^{10}$ et $R^{11}$ représentent chacun un radical alkyle inférieur, avec une cétone de formule

$$R^2-\underset{\underset{O}{\|}}{C}-CH_2-R^3$$

dans laquelle $R^2$ est un radical alkyle inférieur et $R^3$ est l'hydrogène ou un radical alkyle inférieur conformément au procédé à l'indole de Fischer;

(d) lorsqu'un composé de formule I dans laquelle $R^1$ et $R^3$ représentent chacun l'hydrogène ou un radical alkyle inférieur, $R^2$ est un radical alkyle inférieur, $R^4$ est un groupe $NR^6R^7$ dans lequel $R^6$ est l'hydrogène et $R^7$ est l'hydrogène ou un radical alkyle inférieur et $R^5$ est l'hydrogène, ou bien $R^4$ est l'hydrogène et $R^5$ est un groupe $NR^6R^7$ dans lequel $R^6$ est l'hydrogène et $R^7$ est l'hydrogène ou un radical alkyle inférieur, est désiré, hydrogénation d'un composé correspondant de formule XIV

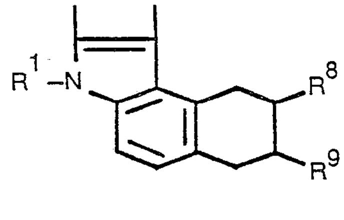

(XIV)

dans laquelle $R^1$ et $R^3$ représentent chacun l'hydrogène ou un radical alkyle inférieur, $R^2$ est un radical alkyle inférieur, $R^8$ est un group $NR^{10}R^{11}$ dans lequel $R^{10}$ est un radical benzyle et $R^{11}$ est un radical

benzyle ou alkyle inférieur et $R^9$ est l'hydrogène, ou bien $R^8$ est l'hydrogène et $R^9$ est un groupe $NR^{10}R^{11}$ dans lequel $R^{10}$ est un radical benzyle et $R^{11}$ est un radical benzyle ou alkyle inférieur;

(e) lorsqu'un composé de formule I dans laquelle $R^1$ et $R^2$ représentent l'hydrogène, $R^3$ est un radical alkyle inférieur, $R^4$ est un groupe $NR^6R^7$ dans lequel $R^6$ et $R^7$ représentent chacun un radical alkyle inférieur et $R^5$ est l'hydrogène, ou bien $R^4$ est l'hydrogène et $R^5$ est un groupe $NR^6R^7$ dans lequel $R^6$ et $R^7$ représentent chacun un radical alkyle inférieur, est désiré, décarboxylation d'un composé correspondant de formule XVI

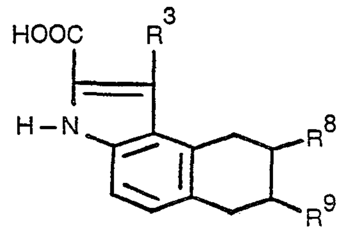

(XVI)

dans laquelle $R^3$ est un radical alkyle inférieur, $R^8$ est un groupe $NR^{10}R^{11}$ dans lequel $R^{10}$ et $R^{11}$ représentent chacun un radical alkyle inférieur et $R^9$ est l'hydrogène, ou bien $R^8$ est l'hydrogène et $R^9$ est un groupe $NR^{10}R^{11}$ dans lequel $R^{10}$ et $R^{11}$ sont chacun un radical alkyle inférieur;

(f) lorsqu'un composé de formule I dans laquelle $R^1$ est l'hydrogène ou un radical alkyle inférieur, $R^2$ est l'hydrogène, $R^3$ est un radical alkyle inférieur, $R^4$ est un groupe $NR^6R^7$ dans lequel $R^6$ est l'hydrogène et $R^7$ est l'hydrogène ou un radical alkyle inférieur et $R^5$ est l'hydrogène, ou bien $R^4$ est l'hydrogène et $R^5$ est un groupe $NR^6R^7$ dans lequel $R^6$ est l'hydrogène et $R^7$ est l'hydrogène ou un radical alkyle inférieur, est désiré, hydrogénation d'un composé correspondant de formule XVII

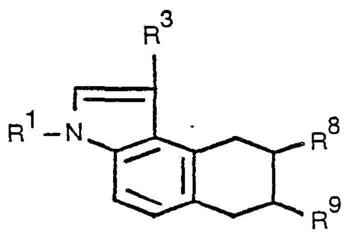

(XVII)

dans laquelle $R^1$ est l'hydrogène ou un radical alkyle inférieur, $R^3$ est un radical alkyle inférieur, $R^8$ est un groupe $NR^{10}R^{11}$ dans lequel $R^{10}$ est un radical benzyle et $R^{11}$ est un radical benzyle ou alkyle inférieur et $R^9$ est l'hydrogène, ou bien $R^8$ est l'hydrogène et $R^9$ est un groupe $NR^{10}R^{11}$ dans lequel $R^{10}$ est un radical benzyle et $R^{11}$ est un radical benzyle ou alkyle inférieur;

(g) lorsqu'un composé de formule I dans laquelle $R^1$ est un radical alkyle inférieur, $R^2$ et $R^3$ représentent chacun l'hydrogène ou un radical alkyle inférieur, $R^4$ est un groupe $NR^6R^7$ dans lequel $R^6$ et $R^7$ représentent chacun un radical alkyle inférieur et $R^5$ est l'hydrogène, ou bien $R^4$ est l'hydrogène, et $R^5$ est un groupe $NR^6R^7$ dans lequel $R^6$ et $R^7$ représentent chacun un radical alkyle inférieur, est désiré, alkylation du composé correspondant de formule I dans laquelle $R^1$ est l'hydrogène, $R^2$ et $R^3$ sont chacun l'hydrogène ou un radical alkyle inférieur, $R^4$ est un groupe $NR^6R^7$ dans lequel $R^6$ et $R^7$ représentent chacun un radical alkyle inférieur et $R^5$ est l'hydrogène, ou bien $R^4$ est l'hydrogène et $R^5$ est un groupe $NR^6R^7$ dans lequel $R^6$ et $R^7$ sont chacun un groupe alkyle inférieur, et

(h) lorsqu'un sel d'addition d'acide thérapeutiquement acceptable d'un composé de formule I est désiré, réaction du composé de formule I avec un acide thérapeutiquement acceptable.

2. Procédé suivant la revendication 1 pour la préparation d'un composé de formule I, dans laquelle $R^1$, $R^2$ et $R^3$ représentent chacun l'hydrogène ou un radical alkyle inférieur ayant 1 à 3 atomes de carbone, $R^4$ est un groupe $NR^6R^7$ dans lequel $R^6$ et $R^7$ représentent chacun l'hydrogène ou un radical alkyle inférieur ayant 1 à 3 atomes de carbone et $R^5$ est l'hydrogène, ou bien $R^4$ est l'hydrogène et $R^5$ est un groupe $NR^6R^7$ dans lequel $R^6$ et $R^7$ représentent chacun l'hydrogène ou un radical alkyle inférieur ayant 1 à 3 atomes de carbone, ou d'un sel d'addition d'acide thérapeutiquement acceptable de ce composé.

3. Procédé suivant la revendication 1 pour la préparation d'un composé de formule I, dans laquelle $R^1$, $R^2$ et $R^3$ représentent l'hydrogène, $R^4$ est un groupe $NR^6R^7$ dans lequel $R^6$ et $R^7$ représentent chacun l'hydrogène ou un radical alkyle inférieur ayant 1 à 3 atomes de carbone et $R^5$ est l'hydrogène, ou bien $R^4$ est l'hydrogène et $R^5$ est un groupe $NR^6R^7$ dans lequel $R^6$ et $R^7$ représentent chacun l'hydrogène ou un groupe alkyle inférieur ayant 1 à 3 atomes de carbone, ou d'un sel d'addition d'acide thérapeutiquement acceptabvle de ce composé.

4. Procédé de préparation d'un composé de formule I

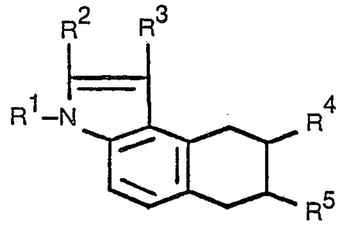

(I)

dans laquelle $R^1$ est l'hydrogène ou un radical alkyle inférieur ayant 1 à 5 atomes de carbone, $R^2$ et $R^3$ représentent l'hydrogène, $R^4$ est un groupe $NR^6R^7$ dans lequel $R^6$ et $R^7$ sont chacun un radical alkyle inférieur ayant 1 à 5 atomes de carbone et $R^5$ est l'hydrogène, ou bien $R^4$ est l'hydrogène et $R^5$ est un groupe $NR^6R^7$ dans lequel $R^6$ et $R^7$ sont chacun un radical alkyle inférieur ayant 1 à 5 atomes de carbone, ou d'un sel d'addition d'acide thérapeutiquement acceptable de ce composé, qui consiste à choisir un mode opératoire dans le groupe des procédés suivants:

(a) lorsqu'un composé de formule I dans laquelle $R^1$, $R^2$ et $R^3$ sont l'hydrogène, $R^4$ est un groupe $NR^6R^7$ dans lequel $R^6$ et $R^7$ sont chacun un radical alkyle inférieur ayant 1 à 5 atomes de carbone et $R^5$ est l'hydrogène, ou bien $R^4$ est l'hydrogène et $R^5$ est un groupe $NR^6R^7$ dans lequel $R^6$ et $R^7$ sont chacun un radical alkyle inférieur ayant 1 à 5 atomes de carbone, est désiré, réduction d'un composé correspondant de formule X

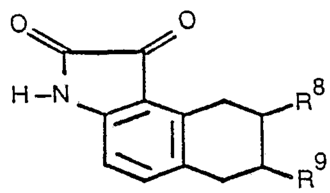

(X)

dans laquelle $R^8$ est un groupe $NR^{10}R^{11}$ où $R^{10}$ et $R^{11}$ sont chacun un radical alkyle inférieur et $R^9$ est l'hydrogène, ou bien $R^8$ est l'hydrogène et $R^9$ est un groupe $NR^{10}R^{11}$ dans lequel $R^{10}$ et $R^{11}$ sont chacun un radical alkyle inférieur, avec un hydrure métallique complexe;

(b) lorsqu'un composé de formule I dans laquelle $R^1$ est un radical alkyle inférieur, $R^2$ et $R^3$ sont l'hydrogène, $R^4$ est un groupe $NR^6R^7$ dans lequel $R^6$ et $R^7$ sont chacun un radical alkyle inférieur et $R^5$ est l'hydrogène, ou bien $R^4$ est l'hydrogène et $R^5$ est un groupe $NR^6R^7$ dans lequel $R^6$ et $R^7$ sont chacun un radical alkyle inférieur, est désiré, alkylation du composé correspondant de formule I dans laquelle $R^1$, $R^2$ et $R^3$ sont l'hydrogène, $R^4$ est un groupe $NR^6R^7$ dans lequel $R^6$ et $R^7$ sont chacun un radical alkyle inférieur et $R^5$ est l'hydrogène, ou bien $R^4$ est l'hydrogène et $R^5$ est un groupe $NR^6R^7$ dans lequel $R^6$ et $R^7$ sont chacun un radical alkyle inférieur, et

(c) lorsqu'un sel d'addition d'acide thérapeutiquement acceptable d'un composé de formule I est désiré, réaction du composé de formule I avec un acide thérapeutiquement acceptable.

5. Procédé suivant la revendication 4 pour la préparation d'un composé de formule I, dans laquelle $R^1$, $R^2$ et $R^3$ sont l'hydrogène, $R^4$ est un groupe $NR^6R^7$ dans lequel $R^6$ et $R^7$ représentent chacun un radical alkyle inférieur ayant 1 à 3 atomes de carbone et $R^5$ est l'hydrogène, ou bien $R^4$ est l'hydrogène et $R^5$ est un groupe $NR^6R^7$ dans lequel $R^6$ et $R^7$ représentent chacun un radical alkyle inférieur ayant 1 à 3 atomes de carbone, ou d'un sel d'addition d'acide thérapeutiquement acceptable de ce composé.

6. Procédé suivant la revendication 4 pour la préparation d'un composé de formule I, dans laquelle $R^1$ est l'hydrogène ou le radical méthyle; $R^2$, $R^3$ et $R^5$ sont l'hydrogène; et $R^4$ est un groupe $NR^6R^7$ dans lequel $R^6$ et $R^7$ sont des radicaux propyle.

7. Procédé suivant la revendication 4 pour la préparation d'un composé de formule I, dans laquelle $R^1$, $R^2$, $R^3$ et $R^4$ sont des atomes d'hydrogène et $R^5$ est un groupe $NR^6R^7$ dans lequel $R^6$ et $R^7$ sont des radicaux propyle.

8. Procédé suivant la revendication 4 pour la préparation d'un composé de formule I, dans laquelle $R^1$ est l'hydrogène ou le radical méthyle; $R^2$, $R^3$ et $R^5$ sont des atomes d'hydrogène; et $R^4$ est un groupe $NR^6R^7$ dans lequel $R^6$ et $R^7$ sont des radicaux méthyle.

9. Procédé suivant la revendication 1 ou 4 dans lequel l'hydrure de complexe métallique est l'hydrure de lithium et d'aluminium.

10. Procédé de preparation d'une composition pharmaceutique, caractérisé en ce qu'un composé de formule I tel que défini dans la revendication 1 ou un sel thérapeutiquement acceptable de ce composé est mélangé avec un support pharmaceutique et mis sous une forme qui convient à l'administration thérapeutique.

11. Procédé de préparation d'un composé de formule

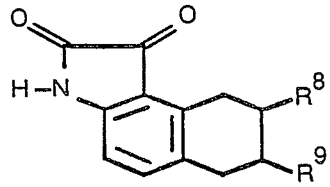

(X)

dans laquelle $R^8$ est un groupe $NR^{10}R^{11}$ où $R^{10}$ et $R^{11}$ représentent chacun un radical benzyle ou alkyle inférieur ayant 1 à 5 atomes de carbone et $R^9$ est l'hydrogène ou bien $R^8$ est l'hydrogène et $R^9$ est un groupe $NR^{10}R^{11}$ dans lequel $R^{10}$ et $R^{11}$ sont chacun un radical benzyle ou alkyle inférieur ayant 1 à 5 atomes de carbone, qui consiste à cycliser, conformément au mode opératoire de la réaction de Sandmeyer de formation d'isatine avec l'isonitrosoacétanilide, un composé de formule IX

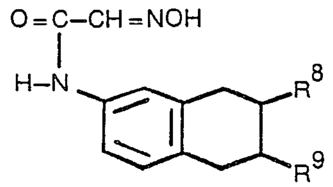

(IX)

dans laquelle $R^8$ et $R^9$ ont les définitions données ci-dessus.